# EUROPEAN PATENT APPLICATION

(11) **EP 4 014 997 A1**
(43) Date of publication of application: **22.06.2022**
(21) Application number: 20383102.9
(22) Date of filing: 16.12.2020
(51) Int. Cl.: A61K 45/00, A61P 43/00

(54) **STARD1 INHIBITORS FOR THE TREATMENT OF LYSOSOMAL DISORDERS**

(71) Applicant: Consejo Superior de Investigaciones Científicas (CSIC), 28006 Madrid (ES); Consorcio Centro de Investigación Biomédica en Red, M.P. (CIBER), 28029 Madrid (ES)
(72) Inventor: GARCIA RUIZ, Mª del Carmen, 08036 Barcelona (ES); TORRES NUÑEZ, Sandra, 08036 Barcelona (ES); SOLSONA I VILARRASA, Estel, 08036 Barcelona (ES); FERNANDEZ-CHECA TORRES, José Carlos, 08036 Barcelona (ES); NUÑEZ POZUELO, Susana, 28029 Madrid (ES)
(74) Representative: Pons

(57) **Abstract**

The invention relates to peptides for use in the treatment and/or diagnostic of lysosomal storage diseases, specifically peptides or proteins that inhibit STARD1 expression levels and subsequently mitochondrial cholesterol levels, and their use in the treatment of lysosomal storage diseases.

SEQUENCE LISTING

<110> Consejo Superior de Investigaciones Científicas, CSIC
      Consorcio Centro de Investigación Biomédica en Red, M.P.
      (CIBER)

<120> STARD1 inhibitors for the treatment of lysosomal disorders

<130> EP1641.1597

<160> 11

<170> BiSSAP 1.3.6

<210> 1
<211> 285
<212> PRT
<213> Homo sapiens


<400> 1

<210> 2
<211> 285
<212> PRT
<213> Homo sapiens

<400> 2

<210> 3
<211> 249
<212> PRT
<213> Homo sapiens

<220>
<221> NON_TER
<222> 1
<223> The first residue of the displayed protein sequence may not
      correspond to the true N-terminus of the complete protein
      sequence

<400> 3
Xaa Leu Arg Gln Gln Ala Val Met Ala Ile Ser Gln Glu Leu Asn Arg
1               5                   10                  15
Arg Ala Leu Gly Gly Pro Thr Pro Ser Thr Trp Ile Asn Gln Val Arg
            20                  25                  30
Arg Arg Ser Ser Leu Leu Gly Ser Arg Leu Glu Glu Thr Leu Tyr Ser
        35                  40                  45
Asp Gln Glu Leu Ala Tyr Leu Gln Gln Gly Glu Glu Ala Met Gln Lys
    50                  55                  60
Ala Leu Gly Ile Leu Ser Asn Gln Glu Gly Trp Lys Lys Glu Ser Gln
65                  70                  75                  80

Gln Asp Asn Gly Asp Lys Val Met Ser Lys Val Val Pro Asp Val Gly
                85                  90                  95
Lys Val Phe Arg Leu Glu Val Val Val Asp Gln Pro Met Glu Arg Leu
            100                 105                 110
Tyr Glu Glu Leu Val Glu Arg Met Glu Ala Met Gly Glu Trp Asn Pro
        115                 120                 125
Asn Val Lys Glu Ile Lys Val Leu Gln Lys Ile Gly Lys Asp Thr Phe
    130                 135                 140
Ile Thr His Glu Leu Ala Ala Glu Ala Ala Gly Asn Leu Val Gly Pro
145                 150                 155                 160
Arg Asp Phe Val Ser Val Arg Cys Ala Lys Arg Arg Gly Ser Thr Cys
                165                 170                 175
Val Leu Ala Gly Met Ala Thr Asp Phe Gly Asn Met Pro Glu Gln Lys
            180                 185                 190
Gly Val Ile Arg Gly Gly Cys Pro Arg Ala Ser Ser Thr Arg Ser Cys
        195                 200                 205
Pro Arg Pro Arg Trp Ile Leu Pro Thr Thr Cys Ala Ser Ala Trp Ser
    210                 215                 220
Pro Thr Leu Pro Leu Lys Pro Gly Val Glu Asp Gln Pro Ala Val Pro
225                 230                 235                 240
Asn Cys Ala Gln Leu His Trp Tyr Thr
                245

<210> 4
<211> 48
<212> PRT
<213> Homo sapiens


<400> 4
Met Gln Lys Ala Leu Gly Ile Leu Ser Asn Gln Glu Gly Trp Lys Lys
1               5                   10                  15
Glu Ser Gln Gln Asp Asn Gly Asp Lys Val Met Ser Lys Val Val Pro
            20                  25                  30
Asp Val Gly Lys Val Phe Arg Leu Glu Val Val Val Asp Gln Pro Met
        35                  40                  45



<210> 5
<211> 2328
<212> DNA
<213> Homo sapiens


<220>
<223> ENA|BC016481|BC016481.1 Homo sapiens N-acylsphingosine
      amidohydrolase (acid ceramidase) 1

<400> 5
ctggagtccg gggagtggcg ttggctgcta gagcgatgcc gggccggagt tgcgtcgcct      60

tagtcctcct ggctgccgcc gtcagctgtg ccgtcgcgca gcacgcgccg ccgtggacag     120

aggactgcag aaaatcaacc tatcctcctt caggaccaac gtacagaggt gcagttccat     180

ggtacaccat aaatcttgac ttaccaccct acaaaagatg gcatgaattg atgcttgaca     240

aggcaccaat gctaaaggtt atagtgaatt ctctgaagaa tatgataaat acattcgtgc     300

caagtggaaa agttatgcag gtggtggatg aaaaattgcc tggcctactt ggcaactttc     360

ctggcccttt tgaagaggaa atgaagggta ttgccgctgt tactgatata cctttaggag     420

agattatttc attcaatatt ttttatgaat tatttaccat ttgtacttca atagtagcag 480

aagacaaaaa aggtcatcta atacatggga gaaacatgga ttttggagta tttcttgggt 540

ggaacataaa taatgatacc tgggtcataa ctgagcaact aaaaccttta acagtgaatt 600

tggatttcca aagaaacaac aaaactgtct tcaaggcttc aagctttgct ggctatgtgg 660

gcatgttaac aggattcaaa ccaggactgt tcagtcttac actgaatgaa cgtttcagta 720

taaatggtgg ttatctgggt attctagaat ggattctggg aaagaaagat gccatgtgga 780

tagggttcct cactagaaca gttctggaaa atagcacaag ttatgaagaa gccaagaatt 840

tattgaccaa gaccaagata ttggccccag cctactttat cctgggaggc aaccagtctg 900

gggaaggttg tgtgattaca cgagacagaa aggaatcatt ggatgtatat gaactcgatg 960

ctaagcaggg tagatggtat gtggtacaaa caaattatga ccgttggaaa catcccttct 1020

tccttgatga tcgcagaacg cctgcaaaga tgtgtctgaa ccgcaccagc caagagaata 1080

tctcatttga aaccatgtat gatgtcctgt caacaaaacc tgtcctcaac aagctgaccg 1140

tatacacaac cttgatagat gttaccaaag gtcaattcga aacttacctg cgggactgcc 1200

ctgacccttg tataggttgg tgagcacacg tctggcctac agaatgcggc ctctgagaca 1260

tgaagacacc atctccatgt gaccgaacac tgcagctgtc tgaccttcca aagactaaga 1320

ctcgcggcag gttctctttg agtcaatagc ttgtcttcgt ccatctgttg acaaatgaca 1380

gatctttttt tttcccccta tcagttgatt tttcttattt acagataact tctttagggg 1440

aagtaaaaca gtcatctaga attcactgag ttttgtttca ctttgacatt tggggatctg 1500

gtgggcagtc gaaccatggt gaactccacc tccgtggaat aaatggagat tcagcgtggg 1560

tgttgaatcc agcacgtctg tgtgagtaac gggacagtaa acactccaca ttcttcagtt 1620

tttcacttct acctacatat ttgtatgttt ttctgtataa cagccttttc cttctggttc 1680

taactgctgt taaaattaat atatcattat ctttgctgtt attgacagcg atataatttt 1740

attacatatg attagaggga tgagacagac attcacctgt atatttcttt taatgggcac 1800

aaaatgggcc cttgcctcta aatagcactt tttggggttc aagaagtaat cagtatgcaa 1860

agcaatcttt tatacaataa ttgaagtgtt ccctttttca taattactct acttcccagt 1920

aaccctaagg aagttgctaa cttaaaaaac tgcatcccac gttctgttaa tttagtaaat 1980

aaacaagtca aagacttgtg gaaaatagga agtgaaccca tattttaaat tctcataagt 2040

agcattcatg taataaacag gtttttagtt tgttcttcag attgataggg agttttaaag 2100

aaattttagt agttactaaa attatgttac tgtatttttc agaaatccaa ctgcttatga 2160

aaagtactaa tagaacttgt taacctttct aaccttcacg attaactgtg aaatgtacgt 2220

catttgtgca agaccgtttg tccacttcat tttgtataat cacagttgtg ttcctgacac 2280

tcaataaaca gtcattggaa aaaaaaaaaa aaaaaaaaaa aaaaaaaa 2328

<210> 6
<211> 20
<212> DNA
<213> Artificial Sequence

<220>
<223> Forward primer

<400> 6
caggagcgca ccatcttctt 20

<210> 7
<211> 19
<212> DNA
<213> Artificial Sequence

<220>
<223> Reverse primer

<400> 7
cttgtgcccc aggatgttg 19

<210> 8
<211> 20
<212> DNA
<213> Artificial Sequence

<220>
<223> Forward primer

<400> 8
agttgcgtcg ccttagtcct 20

<210> 9
<211> 20
<212> DNA
<213> Artificial Sequence

<220>
<223> Rever primer

<400> 9
tgcacctctg tacgttggtc 20

<210> 10
<211> 19
<212> DNA
<213> Artificial Sequence

<220>
<223> Forward primer

<400> 10
ttgccgacag gatgcagaa                                                    19

<210> 11
<211> 20
<212> DNA
<213> Artificial Sequence

<220>
<223> REverse primer

<400> 11
gccgatccac acggagtact                                                   20

## Description

The invention relates to peptides for use in the treatment and/or diagnostic of lysosomal storage diseases. Thus, the present invention relates to the technical field of medical preparations containing peptides for the treatment of the metabolic/nervous system diseases.

### BACKGROUND ART

Lysosomal membrane proteins act at several crucial steps of the lysosome life cycle, including lumen acidification, metabolite export, molecular motor recruitment and fusion with other organelles. Lysosomal storage diseases (LSDs) are a group of inherited metabolic disorders that result from defects in lysosomal function. Lysosomes are sacs of enzymes within cells that digest large molecules and pass the fragments on to other parts of the cell for recycling. This process requires several critical enzymes. If one of these enzymes is defective (for example, because of a mutation), the large molecules accumulate within the cell, eventually killing it.

Among the ∼50 known LSDs several are caused by lysosomal membrane protein dysfunction. Niemann-Pick diseases are a subgroup of lipid storage disorders called sphingolipidoses in which harmful quantities of fatty substances, or lipids, accumulate in the spleen, liver, lungs, bone marrow, and brain. In the classic infantile type-A variant, a missense mutation causes complete deficiency of acid sphingomyelinase. Sphingomyelin is a component of cell membrane including the organellar membrane, so the enzyme deficiency blocks degradation of sphingomyelin, resulting in the accumulation of sphingomyelin within lysosomes in the macrophage-monocyte phagocyte lineage. Affected cells become enlarged, secondary to the distention of lysosomes with sphingomyelin and cholesterol.

Three types of Niemann-Pick disease exist. Mutations in the SMPD1 gene which codes for acid sphingomyelinase, cause Niemann-Pick disease types A and B. Mutations in NPC1 or NPC2 cause Niemann-Pick disease, type C (NPC), which affects a protein used to transport lipids, mostly cholesterol.

Niemann-Pick type C (NPC) disease exhibits a wide array of symptoms, ranging from neurological deterioration, liver disease, splenomegaly and ultimately premature death. Besides the mutations in NPC1 or NPC2, disease-causing mutations on quality control pathways involving the lysosome and endoplasmic reticulum (ER) have also been shown to contribute to NPC disease. Defects in NPC1 account for up to 95% of NPC cases and given its role in lysosomal cholesterol egress, the primary biochemical feature of NPC disease translates in the lysosomal accumulation of free cholesterol in brain and liver.

In addition to lysosomes, cholesterol accumulation has also been reported in mitochondrial fractions of affected organs from Npc1^{-/-} mice, a murine model which reproduces many of the pathological features of the human disease. Mitochondrial cholesterol accumulation has emerged as a critical factor in several diseases, including steatohepatitis, cancer or Alzheimer's disease, due in part to the regulation of mitochondrial function and limitation of mitochondrial antioxidant defenses.

The diagnosis of NPC is confirmed by biochemical testing that demonstrates impaired cholesterol esterification and positive filipin staining in cultured fibroblasts. Biochemical testing to detect carrier status is unreliable. Most individuals with NPC exhibit mutations in NPC1; fewer than 20 individuals have been diagnosed with NPC2, caused by mutations in NPC2. Molecular genetic testing of NPC1 and NPC2 detects disease-causing mutations in approximately 94% of individuals with NPC. Despite the advances of diagnosing NPC, there is still a lack of biomarkers which allow to quickly and efficiently diagnose NPC.

Over the past several years the number of treatments available for patients with LSDs has rapidly increased. Haematopoietic stem cell transplantation, enzyme replacement therapy, substrate reduction, and chaperone therapies are currently available, and gene therapies and other treatments are rapidly advancing. Despite remarkable advances, the efficacy of most of these therapies is limited, particularly because the treatments are usually initiated when organ damage has already occurred. To circumvent this limitation, screening in newborn infants for LSDs has been introduced in many countries. However, this screening is complicated by the broad clinical variability of the disorders and the fact that many individuals who will be detected as having an enzyme deficiency will develop symptoms very late or never in their life.

As examples, NPC treatment options have been reported by Liu *et al.* (Liu et al. Proc Natl Acad Sci USA, 106, 2377(2009)) which showed that 2-hydroxypropyl-β-cyclodextrin (HPBCD) intravenously administered to Npc1 gene-defective (Npc1-/-) mice is effective in improving the medical state or prolonging survival, and when HPBCD is directly administered into the brain, the improving effect thereof is increased a few hundred times, compared with systemic administration. This disparity in effects of intravenous vs intracerebral administration is due to the inability of HPBCD to cross the blood brain barrier. Based on the outcome of these fundamental researches, especially approved humanistic use of HPBCD to NPC child patients was granted (intravenous administration and intrathecal administration). As a result of continuation for more than 1 year of intravenous instillation of HPBCD (2500 mg/kg per time, 1 to 3 times per week) to NPC child patients, the certain effect of reduction of splenomegaly and improvement in a brain wave in child patients was obtained, but a nervous symptom saw no improvement as of yet, and severe side effects have been observed.

Furthermore, hydroxypropyl-y-cyclodextrin was disclosed in the document EP3078379A1, where in addition iPS cells reprogramed using the hSendai virus vector which comprise the NPC gene is also disclosed. The document EP3505185A1 discloses inhibitors of the Glycogen synthase kinase 3 (GSK 3) for the treatment of NPC, while the document US2014220114A1 discloses inhibitors of acyl-coenzyme A:cholesterol acyltransferase 1 (ACAT1) for the treatment of NPC.

Biological related therapies have also been proposed as for example the use of progranulin (PRGN) disclosed in the document WO2015119989A1, antibodies that target the protein Tau (EP2625198B1) and antibodies targeting the triggering receptor expressed on myeloid cells 2 (TREM2). Despite the large body of work relating to possible drugs, compounds and biological agents for the treatment of NPC, as of now the only drug approved for treatment of NPC in several countries as Europe, Japan, Canada, Brasil, Turkey and Australia is miglustat (Zavesca), a glucosylceramide synthase inhibitor, which extends the life span of patients, despite increased brain glucosylceramide levels.

Therefore, alternate biomarkers for the diagnosis and alternate targets for the treatment of NPC are required to address the lack of viable options in the diagnosis and treatment of the devastating disease NPC.

### SUMMARY OF THE INVENTION

The authors of the present invention have found that the expression levels of the steroidogenic acute regulatory protein 1 (STARD1) gene are increased in subjects suffering from lysosomal storage disorders (LSDs), such as Niemann-Pick C (NPC) disease. The authors analyzed mice where the Niemann-Pick protein type C was mutated (NPC1^{-/-}) and found that mitochondrial membranes where overly loaded with cholesterol. Therefore, the increase in the levels of STARD1 can be used as a biomarker for the diagnostic of LSDs. Due to this, the authors analyzed potential mechanisms which could lead to an accumulation of mitochondrial cholesterol. STARD1 has a crucial role in the intramitochondrial cholesterol trafficking from the outer membrane to inner mitochondrial membrane for metabolism. Therefore, the authors analyzed the levels of expression of the gene STARD1 and found these to be increased.

Said increase in the levels of expression STARD1 correlate with the accumulation of cholesterol in mitochondria. The authors of the present invention further found that decreasing the levels of STARD1 resulted in a decrease of the levels of cholesterol in the mitochondria membranes. As the accumulation of mitochondrial cholesterol is a critical factor in diseases like steatohepatitis, cancer and Alzheimer's disease, the authors tested if STARD1 could also have a function in LSD. The authors found that inhibiting STARD1 leads to the reduction of cholesterol in mitochondrial membranes and improves the performance and oxidative stress of fibroblasts of patients with NPC. Hence, the present invention opens up a new therapeutic window to patients of LSDs.

In view of the aforementioned, a first aspect of the present invention relates to a steroidogenic acute regulatory protein 1 (STARD1) inhibitor, from here onwards the "inhibitor of the invention", for use in medicine, from here onwards the medical use of the invention. Another aspect of the invention relates to a STARD1 inhibitor for use in the treatment of LSD in a subject, from here onwards the treatment use of the invention.

STARD1 gene (Ensembl accession number: ENSG00000147465), also known as StAR gene, codifies for 4 transcripts (splice variants) which code for three distinct protein isoforms (SEQ ID NO: 2 to SEQ ID NO: 4) as shown in the table 1 below.

Table 1: Transcripts and proteins corresponding to the gene STARD1 ENSG00000147465, Human (GRCh38.p13), located at Chromosome 8: 38,142,700-38,150,992 reverse strand. GRCh38:CM000670.2.

| **Name** | **SEQ ID** | **Transcript ID Ensembl** | **Isoform of STARD1 protein UniProtKB ID** |
|---|---|---|---|
| STAR-201 | SEQ ID NO: 2 | ENST00000276449.9 | P49675, Q6IBK0 |
| STAR-204 | SEQ ID NO: 3 | ENST00000522050.1 | H0YB94 |
| STAR-203 | SEQ ID NO: 4 | ENST00000521236.1 | E5RH12 |
| STAR-202 | - | ENST00000520114.1 | No protein |

SEQ ID NO: 2











SEQ ID NO: 3
XLRQQAVMAISQELNRRALGGPTPSTWINQVRRRSSLLGSRLEETLYSDQELAYLQQGEE
AMQKALGILSNQEGWKKESQQDNGDKVMSKVVPDVGKVFRLEVVVDQPMERLYEELVERM
EAMGEWNPNVKEIKVLQKIGKDTFITHELAAEAAGNLVGPRDFVSVRCAKRRGSTCVLAG
MATDFGNMPEQKGVIRGGCPRASSTRSCPRPRWILPTTCASAWSPTLPLKPGVEDQPAVP
NCAQLHWYT

SEQ ID NO: 4
MQKALGILSN QEGWKKESQQ DNGDKVMSKV VPDVGKVFRL EVVVDQPM

The STARD1 protein is partly located in mitochondria and synthesized as a conformationally labile precursor with an N-terminal mitochondrial targeting sequence on cytoplasmic ribosomes. By virtue of its so called START domain, STARD1 stoichiometrically binds cholesterol within the cytoplasm and shuttles the hydrophobic cargo through both mitochondrial membranes and the enclosed intermembrane space. The depletion of STARD1 gene has been associated with several diseases including lipoid congenital adrenal hyperplasia and dose sensitive sex reversal while the overexpression or amplification of STARD1 gene has been associated with cancer, especially breast cancer, and Alzheimer's disease. The present invention, further links increased levels of expression of STARD1 to LSDs.

The present invention comprises the use of a STARD1 inhibitor. In the present invention, the term "STARD1 inhibitor" refers to any molecule capable of completely or partially inhibiting the STARD1 gene expression, both by preventing the expression product of said gene from being produced (interrupting the STARD1 gene transcription, blocking the translation of the mRNA coming from the STARD1 gene expression and/or promoting the premature degradation of the mRNA originating from the STARD1 gene), by directly inhibiting the STARD1 protein transporting activity and by promoting/targeting the STARD1 protein degradation by targeting it to the ubiquitin-proteasome pathway or to lysosomal proteolysis. Methods for decreasing/abrogating the expression of the gene encoding the STARD1 protein include, without being limited to, editing technologies such as CRISPR/cas9 or Cas9 nickase technology.

STARD1 inhibitors can be identified using methods based on the capacity of the so-called inhibitor to reduced/abrogate the level of available cellular protein, which can be assessed using Western blotting with antibodies against STARD1, as exemplified in the examples of the present invention.

By way of non-limiting illustration, STARD1 inhibitors suitable for use in the present invention include chemical compounds, antisense oligonucleotides, interference RNAs (siRNAs), catalytic RNAs or specific ribozymes and inhibitory antibodies. Thus, in a preferred embodiment the STARD1 inhibitor is selected from the group consisting of a chemical compound, a STARD1 specific siRNA, a STARD1 specific antisense oligonucleotide, a STARD1 specific ribozyme, a STARD1 specific antibody, and a protein regulating the STARD1 expression.

### Antisense Oligonucleotides

An additional aspect of the invention relates to the use of isolated "antisense" nucleic acids to inhibit expression, for example, for inhibiting transcription and/or translation of a nucleic acid which encodes STARD1, the activity of which is to be inhibited. The antisense nucleic acids can be bound to the target potential of the drug by means of conventional base complementarity or, for example, in the case of binding to double stranded DNA through specific interaction in the large groove of the double helix. Generally, these methods refer to a range of techniques generally used in the art and they include any method which is based on the specific binding to oligonucleotide sequences.

An antisense oligonucleotide can be distributed, for example, as an expression plasmid which, when it is transcribed in cell, produces RNA complementary to at least one unique part of the cellular mRNA encoding STARD1. Alternatively, the antisense oligonucleotide is an oligonucleotide probe generated *ex vivo* which, when introduced into the cell, produces inhibition of gene expression hybridizing with the mRNA and/or gene sequences of a target nucleic acid. Such oligonucleotide probes are preferably modified oligonucleotides which are resistant to endogenous nucleases, for example, exonucleases and/or endonucleases and are therefore stable in vivo. Examples of nucleic acid molecules for use thereof as antisense oligonucleotides are DNA analogs of phosphoramidate, phosphothionate and methylphosphonate.

With respect to the antisense oligonucleotide, the oligodeoxyribonucleotide regions derived from the starting site of the translation, for example, between -10 and +10 of the target gene are preferred. The antisense approximations involve the oligonucleotide design (either DNA or RNA) that are complementary to the mRNA encoding the target polypeptide. The antisense oligonucleotide will be bound to the transcribed mRNA and translation will be prevented.

The oligonucleotides which are complementary to the 5' end of the mRNA, for example the non-translated 5' sequence up to and including the start codon AUG must function in the most efficient manner to inhibit translation. Nevertheless, it has been shown that the sequences complementary to the non-translated 3' sequences of the mRNA are also efficient for inhibiting mRNA translation. Therefore, complementary oligonucleotides could be used at the non-translated 5' or 3' regions, non-coding regions of a gene in an antisense approximation to inhibit the translation of that mRNA. The oligonucleotides complementary to the non-translated 5' region of the mRNA must include the complement of the start codon AUG. The oligonucleotides complementary to the coding region of the mRNA are less efficient translation inhibitors but they could also be used according to the invention. If they are designed to hybridize with the 5' region, 3' region or the coding region of the mRNA, the antisense nucleic acids must have at least six nucleotides long and preferably have less than approximately 100 and more preferably less than approximately 50, 25, 17 or 10 nucleotides long.

Preferably, *in vitro* studies are performed first to quantify the capacity of the antisense oligonucleotides for inhibiting gene expression. Preferably these studies use controls which distinguish between antisense gene inhibition and non-specific biological effects of the oligonucleotides. Also, preferably, these studies compare the levels of target RNA or protein with that of an internal control of RNA or protein. The results obtained using the antisense oligonucleotides can be compared with those obtained using a control oligonucleotide. Preferably the control oligonucleotide is approximately of the same length as the oligonucleotide to be assayed and the oligonucleotide sequence does not differ from the antisense sequence more than it is deemed necessary to prevent the specific hybridization to the target sequence.

The antisense oligonucleotide can be a single or double stranded DNA or RNA or chimeric mixtures or derivatives or modified versions thereof. The oligonucleotide can be modified in the base group, the sugar group or the phosphate backbone, for example, to improve the stability of the molecule, its hybridization capacity, etc. The oligonucleotide may include other bound groups, such as peptides (for example, for directing them to the receptors of the host cells), agents for facilitating transport through the cell membrane or the blood-brain barrier, and intercalating agents. For this purpose, the oligonucleotide can be conjugated to another molecule, for example, a peptide, a transporting agent, hybridization triggered cleaving agent, etc.

The antisense oligonucleotides may comprise at least one modified base. The antisense oligonucleotide may also comprise at least a modified sugar group selected from the group including but not limited to arabinose, 2-fluoroarabinose, xylulose, and hexose. The antisense oligonucleotide may also contain a backbone similar to a neutral peptide. Such molecules are known as peptide nucleic acid (PNA) oligomers. In yet another embodiment, the antisense oligonucleotide comprises at least one modified phosphate backbone. In yet another embodiment, the antisense oligonucleotide is an alpha-anomeric oligonucleotide.

While antisense oligonucleotides complementary to the coding region of the target mRNA sequence can be used, those complementary to the transcribed non translated region can also be used.

In some cases, it may be difficult to reach the sufficient intracellular concentrations of the antisense to suppress the endogenous mRNA translation. Therefore, a preferred approximation uses a recombinant DNA construct in which the antisense oligonucleotide is placed under the control of a strong pol III or pol II promoter.

Alternatively, the target gene expression can be reduced by directing deoxyribonucleotide sequences complementary to the gene regulating region (i.e., the promoter and/or enhancers) to form triple helix structures preventing gene transcription in the target cells in the body. In certain embodiments, the antisense oligonucleotides are antisense morpholines.

### siRNA

Small interference RNA or siRNA are agents which are capable of inhibiting the expression of a target gene by means of RNA interference. A siRNA can be chemically synthesized, can be obtained by means of *in vitro* transcription or can be synthesized *in vivo* in the target cell. Typically, the siRNA consists of a double stranded RNA between 15 and 40 nucleotide long and may contain a 3' and/or 5' protruding region of 1 to 6 nucleotides. The length of the protruding region is independent of the total length of the siRNA molecule. The siRNA acts by means of degrading or silencing the target messenger after transcription.

The siRNAs of the invention are substantially homologous to the mRNA of the STARD1 protein encoding gene or to the gene sequence which encodes said protein. "Substantially homologous" is understood as having a sequence which is sufficiently complementary or similar to the target mRNA such that the siRNA is capable of degrading the latter through RNA interference. The siRNA suitable for causing said interference include siRNA formed by RNA, as well as siRNA containing different chemical modifications such as: (i) siRNA in which the bonds between the nucleotides are different than those appear in nature, such as phosphorothionate bonds; (ii) Conjugates of the RNA strand with a functional reagent, such as a fluorophore; (iii) Modifications of the ends of the RNA strands, particularly of the 3' end by means of the modification with different hydroxyl functional groups in 2' position; (iv) Nucleotides with modified sugars such as 0-alkylated residues on 2' position like 2'-O-methylribose or 2'-O-fluororibose; or (v) Nucleotides with modified bases such as halogenated bases (for example 5-bromouracil and 5-iodouracil), alkylated bases (for example 7-methylguanosine).

The siRNA can be used in the form of a double stranded RNA with the aforementioned characteristics. Alternatively, the use of vectors containing the sense and antisense strand sequence of the siRNA is possible under the control of suitable promoters for the expression thereof in the cell of interest.

Vectors suitable for expressing siRNA are those in which the two DNA regions encoding the two strands of siRNA are arranged in tandem in one and the same DNA strand separated by a spacer region which, upon transcription, forms a loop and wherein a single promoter directs the transcription of the DNA molecule giving rise to the so-called short hairpin RNA (shRNA).

The siRNA can be generated intracellularly from the shRNA characterized in that the antiparallel strands forming the siRNA are connected by a loop or hairpin region. The shRNAs can be encoded by plasmids or viruses, particularly retroviruses, and are under the control of a promoter. Promoters suitable for expressing shRNA are those indicated in the paragraph above for expressing siRNA.

The siRNA and shRNA of the invention can be obtained using a series of techniques known by the person skilled in the art. The region of the nucleotide sequence taken as a basis for designing the siRNA is not limiting and it may contain a region of the coding sequence (between the start codon and the end codon) or it may alternatively contain sequences of the non-translated 5' or 3' region preferably between 25 and 50 nucleotides long and in any position in 3' direction position with respect to the start codon. One way of designing an siRNA involves the identification of the AA(N19)TT motifs wherein N can be any nucleotide in the LRRK2 gene sequence, and the selection of those having a high G/C content. If said motif is not found, it is possible to identify the NA(N21) motif wherein N can be any nucleotide.

### DNA Enzymes

The invention also contemplates the use of DNA enzymes to inhibit the expression of the STARD1 gene of the invention. DNA enzymes incorporate some of the mechanistic features of both antisense and ribozyme technologies. DNA enzymes are designed such that they recognize a particular target nucleic acid sequence similar to the antisense oligonucleotide, nevertheless like the ribozyme they are catalytic and specifically cleave the target nucleic acid.

### Ribozymes

Ribozyme molecules designed for catalytically cleaving transcription products of a target mRNA to prevent the translation of the mRNA which encodes STARD1, the activity of which is to be inhibited, can also be used. Ribozymes are enzymatic RNA molecules capable of catalyzing specific RNA cleaving. The mechanism of ribozyme action involves a specific hybridization of a ribozyme molecule sequence to a complementary target RNA followed by an endonucleolytic cleavage event. The composition of the ribozyme molecules preferably includes one or more sequences complementary to the target mRNA and the well-known sequence responsible for cleaving the mRNA or a functionally equivalent sequence. Examples of ribozymes to be used in the present invention include hammer-head ribozymes and endoribonuclease RNA (hereinafter "Cech type ribozymes").

The ribozymes can be formed by modified oligonucleotides (for example to improve the stability, targeting, etc.) and they should be distributed to cells expressing the target gene *in vivo.* A preferred distribution method involves using a DNA construct which "encodes" the ribozyme under the control of a strong constitutive pol III or pol II promoter such that the transfected cells will produce sufficient amounts of the ribozyme to destroy the endogenous target messengers and to inhibit translation. Since the ribozymes are catalytic, unlike other antisense molecules, a low intracellular concentration is required for its efficiency.

### Inhibitory antibodies

In the context of the present invention, "inhibitory antibody" is understood as any antibody capable of binding specifically to the STARD1 protein and inhibiting one or more of the functions of said protein. The antibodies can be prepared using any of the methods which are known by the person skilled in the art, some of which have been mentioned above. Thus, the polyclonal antibodies are prepared by means of immunizing an animal with the protein, or a fragment of said protein, to be inhibited. The monoclonal antibodies are prepared using the method described by Kohler and Milstein et al. 1975 (Nature 256: 495-497). In the context of the present invention, suitable antibodies include intact antibodies comprising a variable antigen binding region and a constant region, "Fab", "F(ab')2" and "Fab"', Fv, scFv fragments, diabodies and bispecific antibodies. Once antibodies with STARD1 protein binding capacity are identified, those capable of inhibiting the activity of this protein will be selected using an inhibitory agent identification assay. STARD1 inhibitory antibodies are commercially available, such as the anti-STARD1 monoclonal antibodies produced in mouse and rabbit (ab58013 and ab133657 respectively, from Abcam), the anti-STARD1 polyclonal antibodies produced in rabbit (ab96637 and ab180804 from Abcam), the anti-STARD1 antibodies produced in rabbit (AP5465PU-N and TA350457 from Origene Technologies), etc.

### Inhibitory chemical compounds.

Chemical compounds which work as a STARD1 inhibitor are able to prevent the transport of cholesterol to the mitochondrial inner membrane for metabolism, for example by occupying the cargo binding site of STARD1.

### Inhibitory proteins

As used herein, the term "inhibitory protein" refers to those proteins capable of binding to the STARD1 protein and inhibiting its activity as well as proteins that can interrupt, inhibit, stop or regulate STARD1 expression, i.e., the formation of functional products such as mRNA and protein, from the STARD1 gene. In addition, the term "inhibitory proteins" also includes fragments of proteins wherein said fragments have the STARD1 inhibitory effect. "Inhibitory proteins" can achieve the inhibiting STARD1 effect by inhibiting STARD1 transporting function, such as blocking access to STARD1 transport subtract or inhibiting STARD1 translocation from outer to inner membrane, by sequestering STARD1 from its normal location or stopping STARD1 to locate at its normal location, by promoting degradation of STARD1 protein by the ubiquitin-proteasome pathway or lysosomal pathway, by downregulating or blocking STARD1 gene transcription or downregulation or blocking STARD1 mRNA translation. In a preferred embodiment the STARD1 inhibitor is a protein regulating the STARD1 expression. In another preferred embodiment the protein regulating the STARD1 expression is an acid ceramidase (ACDase) enzyme or a functionally equivalent variant or fragment thereof.

The term "acid ceramidase" or its abbreviature ("ACDase") as used herein refers to an acylsphingosine deacylase or glycosphingolipid ceramide deacylase (EC 3.5.1.23) which cleaves fatty acids from ceramide, producing sphingosine (SPH). Ceramidases comprise a heterogeneous family of enzymes whose main function in cells is to break down ceramides to SPH (2-amino-4-octadecene-1, 3-diol). Ceramidase enzymes are classified into three groups (acid, neutral, and alkaline enzymes) based on optimum pH and primary structure. Ceramidase activity can be determined as set forth by Bedia et al., Chembiochem 2007 Apr 16;8(6):642-8.

The term "functionally equivalent variant" refers to a protein (or polypeptide) comprising one or more alterations, such as substitutions, insertions, deletions and/or truncations of one or more specific amino acid residues at one or more specific positions in the protein, and having the same function that the original protein. Thus, variants of the ACDase enzyme protein of the invention may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the protein is an alternative splice variant of the proteins of the present invention and/or (iv) "fragments" of the protein. The "fragments" include proteins generated via proteolytic cleavage (including multi-site proteolysis) of an original sequence. Variants are deemed to be within the scope of those skilled in the art from the teaching herein. The term "functionally equivalent fragment" means a protein or a domain thereof having one or more (e.g., several) amino acids absent from the amino and/or carboxyl terminus of the amino acid sequence, wherein the fragment has the same activity than the whole protein.

In the context of the present invention, it is considered that peptides or polypeptides or proteins with an identity of 70% with SEQ ID NO: 1 are functionally equivalent variants of the SEQ ID NO: 1 and maintain the same properties as the sequence to which they refer, i.e., they show STARD1 inhibitory activity. Therefore, in a preferred embodiment the ACDase enzyme comprises an amino acid sequence having an identity of at least 70% with the sequence SEQ ID NO: 1.

SEQ ID NO: 1







The term "identity" or "sequence identity" as used herein refers to the degree of similarity between two nucleotide or amino acid sequences obtained by aligning the two sequences, i.e. the proportion of identical nucleotide or amino acids between two compared nucleotide sequences or polypeptides/proteins along their full-length sequence. Depending on the number of common residues between the aligned sequences, a different degree of identity, expressed as a percentage, will be obtained. The degree of identity between two amino acids or nucleotides sequences may be determined by conventional methods, for example, by standard sequence alignment algorithms known in the state of the art, such as BLAST [Altschul S.F. et al. Basic local alignment search tool. J Mol Biol. 1990 Oct 5; 215(3): 403-10]. The BLAST programs, for example, BLASTN, BLASTX, and T BLASTX, BLASTP and TBLASTN, are in the public domain at *The National Center for Biotechonology Information* (NCBI) website. The skilled person in the art understands that mutations in the nucleotide sequence of genes that lead to conservative amino acid substitutions at non-critical positions for the functionality of the protein are evolutionarily neutral mutations which do not affect its global structure or its functionality.

The present invention relates to a STARD1 inhibitor for use in the treatment of LSD in a subject, preferably NPA, NPB and NPC.

As used herein, the term "treating" (or "treat" or "treatment" or "therapy") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. Furthermore, the "treatment" also refers to a process involving the avoiding of the appearance of symptoms which have not manifested themselves yet, but which will manifest themselves due to an untreated progression of the disorder, condition or disease. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). In the present invention, the condition or disorder to be treated are LSDs. Specifically, the LSD is NPA, NPB and NPC.

The term "lysosomal storage disorders", or its abbreviation "LSDs", as used herein refers to a group of rare inherited metabolic disorders that result from defects in lysosomal function. "Lysosomes" refer to sacs of enzymes within cells that digest large molecules and pass the fragments on to other parts of the cell for recycling. This process requires several critical enzymes. If one of these enzymes is defective due to a mutation, the large molecules accumulate within the cell, eventually killing it. LSDs are caused by lysosomal dysfunction usually as a consequence of deficiency of a single enzyme required for the metabolism of lipids, glycoproteins (sugar-containing proteins), or so-called mucopolysaccharides. The LSDs are generally classified by the nature of the primary stored material involved, and can be broadly broken into the following classification: lipid storage disorders; Sphingolipidoses (including Gaucher's and Niemann-Pick diseases); Gangliosidosis (including Tay-Sachs disease); Leukodystrophies; Mucopolysaccharidoses, (including Hunter syndrome and Hurler disease); Glycoprotein storage disorders; and Mucolipidoses. Therefore, in a preferred embodiment of the medical use of the invention or of the treatment use of the invention, the lysosomal storage disorder is selected from a list consisting of: Farber disease, Krabbe disease, Fabry disease, Schindler disease, GM1 gangliosidosis, GM2 gangliosidosis, GM2 gangliosidosis AB variant, Sandhoff disease, Tay-Sachs, Gaucher disease Type I, Gaucher disease Type II, Gaucher disease Type III, Lysosomal acid lipase deficiency, Niemann-Pick disease Type A, Niemann-Pick disease Type B, Niemann-Pick disease Type C, Metachromatic leukodystrophy, Multiple sulfatase deficiency, Hurler syndrome, Scheie syndrome, Hurler-Scheie syndrome, Hunter syndrome, Sanfilippo syndrome Type A, Sanfilippo syndrome Type B, Sanfilippo syndrome Type C, Sanfilippo syndrome Type D, Morquio syndrome Type A, Morquio syndrome Type B, Maroteaux-Lamy syndrome, Sly syndrome, hyaluronidase deficiency, sialidosis, I-cell disease, phosphotransferase deficiency, mucolipidin 1 deficiency, Santavuori-Haltia disease, Jansky-Bielschowsky disease, Batten-Spielmeyer-Vogt disease, Kufs disease, Neuronal ceroid lipofuscinoses Type 5, Neuronal ceroid lipofuscinoses type 6, Neuronal ceroid lipofuscinoses Type 7, Neuronal ceroid lipofuscinoses Type 8, Neuronal ceroid lipofuscinoses Type 9, Neuronal ceroid lipofuscinoses Type 10, Wolman disease, Alpha-mannosidosis, Beta-mannosidosis, Aspartylglucosaminuria, Fucosidosis, Cystinosis, Pycnodysostosis, Salla disease, Infantile free sialic acid storage disease, Pompe disease and Danon disease. In a further preferred embodiment of the medical use of the invention or the treatment use of the invention, the lysosomal storage disease is selected from a list consisting of: GM1 gangliosidosis, GM2 gangliosidosis, Alpha-mannosidosis, Hurler syndrome, Hunter syndrome, Sanfilippo syndrome Type A, Morquio syndrome Type A, Morquio syndrome Type B, Niemann-Pick type A (NPA), Niemann-Pick type B (NPC B) or Niemann-Pick type C (NPC) disease.

Although the signs and symptoms vary from disease to disease in this group, symptoms occur in each case because of an enzyme deficiency that inhibits the ability of the lysosomes present in each of the body's cells to perform their normal function. The symptoms of lysosomal storage disorders are generally progressive over a period of time. Some lysosomal storage diseases and a few of their characteristic signs and symptoms are as follows:
- Gaucher Disease Types I, II, and III: Gaucher disease is the most common type of lysosomal storage disorder. Researchers have identified three distinct types of Gaucher disease based upon the absence (type I) or presence and extent of (types II and III) neurological complications. Most affected individuals have type I, and they may experience easy bruising, chronic fatigue, and an abnormally enlarged liver and/or spleen (hepatosplenomegaly). Gaucher disease type II occurs in newborns and infants, and is characterized by neurological complications that may include involuntary muscle spasms, difficulty swallowing and the loss of previously acquired motor skills. Gaucher disease type III appears during the first decade of life. Neurological complications may include mental deterioration, an inability to coordinate voluntary movements, and muscle spasms of the arms, legs, or entire body.
- GM2-Gangliosidosis Type I (Tay Sachs Disease): Two main forms of Tay Sachs disease exist: the classic or infantile form and the late-onset form. In individuals with infantile Tay Sachs disease, symptoms typically first appear between three and five months of age. These may include feeding problems, general weakness (lethargy), and an exaggerated startle reflex in response to sudden loud noises. Motor delays and mental deterioration are progressive. In individuals with the late-onset form, symptoms may become apparent anytime from adolescence through the mid-30s. The infantile form often progresses rapidly, resulting in significant mental and physical deterioration. A characteristic symptom of Tay Sachs disease, which occurs in 90 percent of cases, is the development of cherry red spots in the backs of the eyes. Symptoms of late-onset Tay Sachs disease vary widely from case to case. This disorder progresses much more slowly than the infantile form.
- GM2-Gangliosidosis Type II (Sandhoff Disease): The first symptoms of Sandhoff disease typically begin between the ages of three and six months. The disease is clinically indistinguishable from GM2-Gangliosidosis Type I.
- Mucopolysaccharide (MPS) Storage Diseases (Hurler Disease and variants, Hunter, Sanfilippo Types A, B, C, D, Morquio Types A and B, Maroteaux-Lamy and Sly diseases): The MPS diseases are caused by disturbances in the normal breakdown of complex carbohydrates known as mucopolysaccharides. All of the MPS diseases have certain characteristics in common, which include deformities of the bones and joints that interfere with mobility and often cause osteoarthritis, especially of the large, weight-bearing joints. All of the MPS diseases except Sanfilippo disease interfere with growth, causing short stature.
- Alpha-mannosidosis: Alpha-mannosidosis is a lifelong multi-systemic progressive disease, with neuromuscular and skeletal deterioration over decades. The timing of the appearance of symptoms correlates with the severity of the disease. The onset of the most severe form of the disease occurs within the first months of life and involves skeletal abnormalities and intellectual disability, with rapid progression leading to death from primary central nervous system involvement or myopathy. The first decade of life is characterized by the development of hearing impairment, psychomotor delay, recurrent infections, especially upper airway infections, pulmonary infections and acute/serous otitis media infections. Significant changes in a number of facial features may occur, such as: protruding forehead; flattened nasal bridge; small nose; wide mouth; and widely spaced teeth. Muscular weakness or spinal abnormalities can occur due to the build-up of storage materials in the muscle.
- Niemann-Pick disease: Niemann-Pick diseases are an inherited LSD wherein sphingomyelin/cholesterol accumulates in lysosomes. Said accumulation can lead enlargement of the liver and/or spleen enlargement (hepatospenomegaly and/or splenomegaly), accumulation of sphingomyelin in the bones and in the central nervous system (CNS), including cerebellum, basal ganglia, upper brainstem, cerebral cortex and subcortical structures. Symptoms of Niemann-Pick disease are related to the organs where the accumulation of sphingomyelin occurs. Therefore, Niemann-Pick disease symptoms may include reduced appetite, abdominal distension, pain, low levels of platelets in the blood (thrombocytopenia), unsteady gait (ataxia), slurring of speech (dysarthria), difficulty swallowing (dysphagia), abnormal posturing of the limbs, trunk, and face (dystonia), impaired voluntary rapid eye movements (supranuclear gaze palsy), gradual loss of intellectual abilities, causing dementia and seizures, enlarged bone marrow cavities, thinned cortical bone, distortion of the hip bone (coxa vara), sleep inversion, sleepiness during the day and wakefulness at night, sudden loss of muscle tone when the affected patient laughs, prolong jaundice or elevated bilirubin at birth and premature death. Three types of Niemann-Pick diseases exist: Type A (NPA), B (NPB) and C (NPC). NPA and NPB are caused by mutations in the Sphingomyelin phosphodiesterase 1 (SMPD1) gene which leads to a deficiency in the activity of the of the lysosomal enzyme acid sphingomyelinase, that breaks down the lipid sphingomyelin. Mutations in genes Niemann-Pick disease type C 1 or 2 (NPC1 or NPC2), which affects a protein used to transport lipids.

The present invention describes how the accumulation of mitochondrial cholesterol in LSDs can be counteracted by inhibiting STARD1 gene expression or inhibiting its functional products in a subject. The subject to be treated with the STARD1 inhibitor can be any subject as long as the subject comprises mitochondrial cholesterol accumulation.

As used herein, the term "subject" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In a particular embodiment, the subject is a human, man or woman of any age or race.

The subject object of the present invention is characterized by comprising mitochondrial cholesterol accumulation. The term "mitochondrial cholesterol accumulation" as used herein refers to an increase in the cholesterol present in the membranes of mitochondria of any organs, tissues and/or cells. In a preferred embodiment the mitochondrial cholesterol accumulation occurs in the liver and/or brain.

An assay to determine if a given subject suffers from mitochondrial cholesterol accumulation comprises the staining of tissue with perfringolysin O fused with glutathione S-transferase (GST-PFO). In tissues, PFO is able to track cholesterol of outer and inner membranes when these have more than 30% mol of cholesterol and the fusion with GST allows the detection by immunofluorescence techniques widely known by the skilled person in the art.

The STARD1 inhibitor of the present invention may be used in combination with one or more other drugs in the treatment, control, amelioration, prevention of symptoms of LSDs, where the combination of the drugs together is safer or more effective than either drug alone. Thus, in particular embodiment, the inhibitor of the invention is administered in combination with another compound useful for treating LSD. Such other compound(s)/drug(s) may be administered by a route and in an amount commonly used therefore, concomitantly or sequentially with the STARD1 inhibitor. When the STARD1 inhibitor is used concomitantly with one or more other drugs, a pharmaceutical composition in unit dosage form containing such other drugs and the STARD1 inhibitor is preferred. However, the combination therapy may also include therapies in which the STARD1 inhibitor and one or more other drugs are administered on different overlapping schedules. It is also contemplated that when used in combination with one or more other active ingredients, the STARD1 inhibitor and the other active ingredients may be used in lower doses than when each is used singly. Accordingly, the pharmaceutical compositions of the present invention include those that contain one or more other active ingredients, in addition to a STARD1 inhibitor.

Examples of other compounds useful for treating LSDs which may be used in conjunction with STARD1 inhibitor include, without limiting to, alglucerase, a placenta-derived enzyme, imiglucerase, a synthetic form of alglucerase, miglustat a synthetic analogue of D-glucose, Hydroxypropyl-γ-cyclodextrin or GSK3 inhibitors like lithium salt and the compounds CHIR99021, 6-BIO, TDZD-8, curcumin and histone deacetylase (HDAC) inhibitors.

The weight ratio of the STARD1 inhibitor of the present invention to the other active ingredient(s) may be varied and will depend upon the effective dose of each ingredient. Generally, an effective dose of each will be used. Thus, for example, when the STARD1 inhibitor of the present invention is combined with another agent, the weight ratio of the STARD1 inhibitor to the other agent will generally range from about 1000:1 to about 1:1000, or from about 200:1 to about 1:200. Combinations of STARD1 inhibitor and other active ingredients will generally also be within the aforementioned range, but in each case, an effective dose of each active ingredient should be used.

The STARD1 inhibitors of the present invention may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual, buccal or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals the STARD1 inhibitors of the invention are effective for use in humans.

The STARD1 inhibitors of the present invention can be incorporated into pharmaceutical compositions for its administration to the subject. Thus, in a particular embodiment, the inhibitor of the invention is comprised within a pharmaceutical composition. The STARD1 inhibitors of the present invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. Oral tablets may also be formulated for immediate release, such as fast melt tablets or wafers, rapid dissolve tablets or fast dissolve films.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions and the like, containing the compounds of the present invention are employed. Similarly, transdermal patches may also be used for topical administration.

In the treatment, control, amelioration, prevention of symptoms or reduction of risk of LSD, an appropriate dosage level of the STARD1 inhibitor of the invention will generally be about 0.01 to 500 mg per kg patient body weight per day which can be administered in single or multiple doses. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions may be provided in the form of tablets containing 1.0 to 1000 milligrams of the active ingredient, particularly 1.0, 5.0, 10.0, 15.0. 20.0, 25.0, 50.0, 75.0, 100.0, 150.0, 200.0, 250.0, 300.0, 400.0, 500.0, 600.0, 750.0, 800.0, 900.0, and 1,000.0 milligrams of the LRRK2 inhibitor for the symptomatic adjustment of the dosage to the patient to be treated. The compounds may be administered on a regimen of 1 to 4 times per day, or may be administered once or twice per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

As explained previously in the present description, the present invention allows the skilled person in the art to identify LSD patients as well as to determine which patients will benefit from a STARD1-related therapeutic intervention such as STARD1 inhibitors.

Therefore, another aspect of the invention relates to an *in vitro* method for diagnosing lysosomal storage disorder in a subject, or for designing a customized therapy for a subject suffering from lysosomal storage disorder, here onwards the "method of the invention", comprising:
(i) quantifying the expression levels of the nucleotide sequence encoding the STARD1 in a biological sample isolates from the subject, and
(ii) comparing the expression levels obtained in step (i) with a reference level, wherein an increased STARD1 expression levels with respect to the reference level is indicative that the subject suffers from lysosomal storage disorder, or that the therapy to be administered to the subject is based on a STARD1 inhibitor.

The terms "therapy", "lysosomal storage disorder", "subject", "STARD1" and "STARD1 inhibitor" have been explained above in the present description, and these explanations are applicable to the present inventive step.

The term "diagnosing" as used herein refers to the procedure by which a certain disease, nosological entity, syndrome, or any health-disease condition is identified through the analysis of a series of clinical parameters or symptoms characteristic of that disease, and which distinguish it from other diseases with similar clinical presentation. In the present invention the disease to be identified is an LSD, and the clinical parameter is the level of expression of the STARD1 gene. In a preferred embodiment of the method of the invention the LSD to diagnose is select from a list consisting of: Farber disease, Krabbe disease, Fabry disease, Schindler disease, GM1 gangliosidosis, GM2 gangliosidosis, GM2 gangliosidosis AB variant, Sandhoff disease, Tay-Sachs, Gaucher disease Type I, Gaucher disease Type II, Gaucher disease Type III, Lysosomal acid lipase deficiency, Niemann-Pick disease Type A, Niemann-Pick disease Type B, Niemann-Pick disease Type C, Metachromatic leukodystrophy, Multiple sulfatase deficiency, Hurler syndrome, Scheie syndrome, Hurler-Scheie syndrome, Hunter syndrome, Sanfilippo syndrome, Morquio, Maroteaux-Lamy syndrome, Sly syndrome, hyaluronidase deficiency, sialidosis, I-cell disease, phosphotransferase deficiency, mucolipidin 1 deficiency, Santavuori-Haltia disease, Jansky-Bielschowsky disease, Batten-Spielmeyer-Vogt disease, Kufs disease, Neuronal ceroid lipofuscinoses Type 5, Neuronal ceroid lipofuscinoses type 6, Neuronal ceroid lipofuscinoses Type 7, Neuronal ceroid lipofuscinoses Type 8, Neuronal ceroid lipofuscinoses Type 9, Neuronal ceroid lipofuscinoses Type 10, Wolman disease, Alpha-mannosidosis, Beta-mannosidosis, Aspartylglucosaminuria, Fucosidosis, Cystinosis, Pycnodysostosis, Salla disease, Infantile free sialic acid storage disease, Pompe disease and Danon disease. In a further preferred embodiment of the method of the invention, the lysosomal storage disease is selected from a list consisting of: GM1 gangliosidosis, GM2 gangliosidosis, Alpha-mannosidosis, Hurler syndrome, Hunter syndrome, Sanfilippo syndrome Type A, Morquio syndrome Type A, Morquio syndrome Type B, Niemann-Pick type A (NPA), Niemann-Pick type B (NPC B) or Niemann-Pick type C (NPC) disease.

The expression "quantifying the expression levels of the nucleotide sequence encoding the STARD1 in a biological sample isolates from the subject" form step (i) of the method of the invention refers to the procedure of reporting and/or determine the amount of the level of expression of the nucleotide sequence of the gene STARD1, by the detection and/or determination of the amount of the mRNA, or a fragment of the mRNA, cDNA or a fragment of the cDNA, protein or a fragment of the protein encoded by the STARD1 nucleotide sequence. Thus, in a preferred embodiment of the method of the invention the quantification of the expression levels comprises measuring the level of cDNA, the level of mRNA, and/or the level of the protein encoded by said nucleotide sequence.

The terms "mRNA fragment" or "cDNA fragment" as used herein refers to the nucleotide sequence of the STARD1 gene comprising one or more nucleotides absent from the 3' and/or 5' ends with respect to the complete nucleotide sequence of the STARD1 gene.

Likewise, the term "protein fragment" as used herein refers to the amino acid sequence of the STARD1 protein(s) comprising one or more amino acids absent from its terminal amino or terminal carboxyl end with respect to the complete amino acid sequence of the STARD1 protein. In a preferred realization, the STARD1 protein fragment is a fragment of the protein sequence encoded by the sequence selected from the list consisting of SEQ ID NO: 2, SEQ ID NO: 3, SEQ ID NO: 4 or any combination thereof.

Methods for determining the expression levels of a nucleotide sequence are widely known in the state of the art, and any of them can be used in the context of the present invention. The determination of the expression levels of the nucleotide sequence encoding the STARD1 gene may comprise measuring the level of cDNA, the level of mRNA, and/or the level of the protein encoded by said gene. By way of illustration and not limitation, the levels of mRNA of said gene can be quantified by means of using conventional methods, for example, methods comprising the amplification of mRNA and the quantification of the product of the amplification of said mRNA, such as electrophoresis and staining, or alternatively, by means of Southern blot and the use of suitable probes, Northern blot and the use of specific probes of mRNA of the STARD1 gene or of the corresponding cDNA thereof, S1 nuclease mapping, hybridisation, RT-Q-PCR, the micro-arrays and RNAsequencing, etc. If the quantification of the expression of STARD1 gene encoding is going to be carried out from the protein, i.e. the STARD1 protein, then the isolated biological sample of the subject must be treated to extract the proteins. Methods for extracting or isolating proteins are known by the person skilled in the art and they are commercially available. The levels of gene encoding the STARD1 protein can be quantified by means of any conventional method that enables said protein to be detected and quantified in a sample of a subject. By way of illustration and not limitation, the levels of said protein can be quantified, for example, by means of the use of antibodies with the capacity to bind specifically to the STARD1 protein and the subsequent quantification of the complexes formed. Examples of antibodies with the capacity to specifically bind to the STARD1 protein have been disclosed in previous paragraphs.

The expression "comparing the expression levels obtained in step (i) with a reference level" in the method of the invention refers to the process or procedure of relating, examining or verifying the values obtained for the expression level of the STARD1 gene, and equate or verify the values obtained in step a) with the values expected to be obtained for subjects not suffering from the disease and/or not presenting mitochondrial cholesterol accumulation, where, according to the method of the invention, "an increased STARD1 expression levels with respect to the reference level is indicative that the subject suffers from lysosomal storage disorder, or that the therapy to be administered to the subject is based on a STARD1 inhibitor".

The term "reference level" refers to the expression levels of the nucleotide sequence encoding the STARD1 gene in a sample of a subject not suffering from LSD and/or not comprising mitochondrial cholesterol accumulation. In a preferred embodiment of the method of the invention the subject is a mammal, preferably, the mammal is a human.

The method of the invention contemplates the diagnostic as well as the designing of a customized therapy for a subject that suffers from LSD, wherein said customized therapy is based on a STARD1 inhibitor. In a preferred embodiment of the method of the invention, the STARD1 inhibitor is selected from the group consisting of a chemical compound, a STARD1 specific siRNA, a STARD1 specific antisense oligonucleotide, a STARD1 specific ribozyme and a STARD1 specific antibody, and a protein regulating the STARD1 expression.

In another preferred embodiment of the method of the invention, the protein regulating the STARD1 expression is an acid ceramidase (ACDase) enzyme or a functionally equivalent variant or functionally equivalent fragment thereof.

In yet another preferred embodiment of the method of the invention, the ACDase enzyme comprises an amino acid sequence having an identity of at least 70% with the sequence SEQ ID NO: 1.

The terms "chemical compound", "siRNA", antisense oligonucleotide, "ribozyme", "antibody", "protein", "acid ceramidase", "ACDase", "functionally equivalent variant", "functionally equivalent fragment" and "identity" have been explained and exemplified above in the present description, and these explanations and examples are applicable to the present inventive step.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

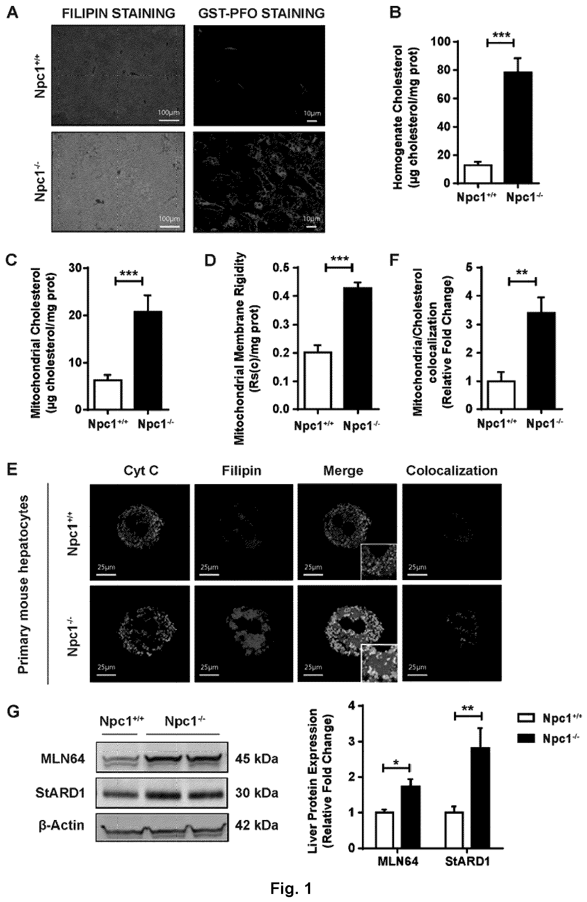

**Figure 1****. Mitochondrial cholesterol trafficking and STARD1 expression in livers of Npc1^{-/-} mice.** Npc1^{+/+} and Npc1^{-/-} mice were sacrificed at 6 weeks of age to isolate mitochondria from liver. (A) Filipin and GST-PFO staining. (B) Homogenate and (C) mitochondrial cholesterol levels by HPLC analyses. (D) Mitochondrial membrane order measured by DPH fluorescence anisotropy. (E) Cytochrome c and Filipin co-staining for confocal microscopy analyses of Npc1^{+/+} and Npc1^{-/-} primary mouse hepatocytes (F) Staining markers colocalization analysis using ImageJ software. (G) Western blot analyses and quantification of the mitochondrial cholesterol carriers STARD1 and MLN64 in liver of 6-weeks old Npc1^{+/+} and Npc1^{-/-} mice. Data are presented as means ± SEM (n>4, Unpaired Student's t-test (two-tailed)).

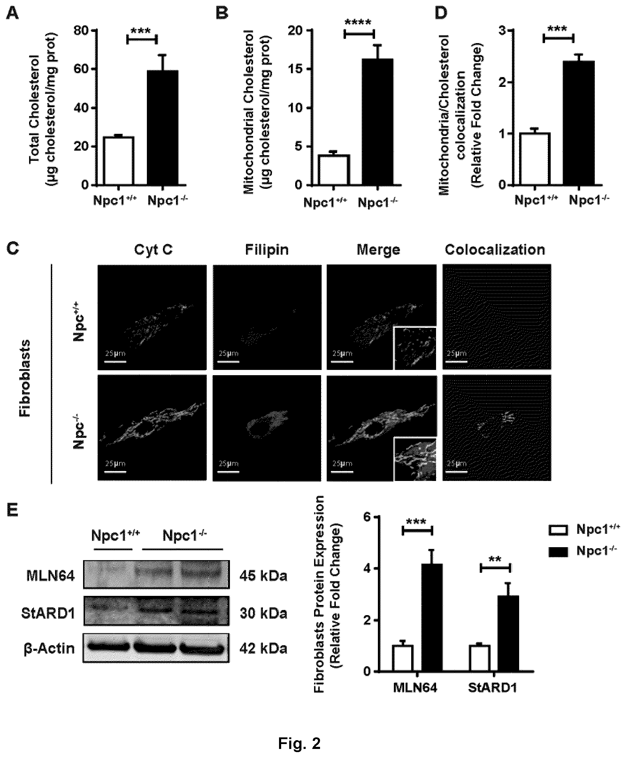

**Figure 2****. Mitochondrial cholesterol trafficking and STARD1 expression in human skin fibroblasts from control subjects or patients with NPC disease.** (A) Homogenate and (B) mitochondrial cholesterol levels by HPLC analyses. (C) Cytochrome c and Filipin co-staining for confocal microscopy analyses (D) Staining markers colocalization analysis using ImageJ software. (E) Western blot analyses and quantification of the mitochondrial cholesterol carriers STARD1 and MLN64. Data are presented as means ± SD (n>5, Unpaired Student's t-test (two-tailed)).

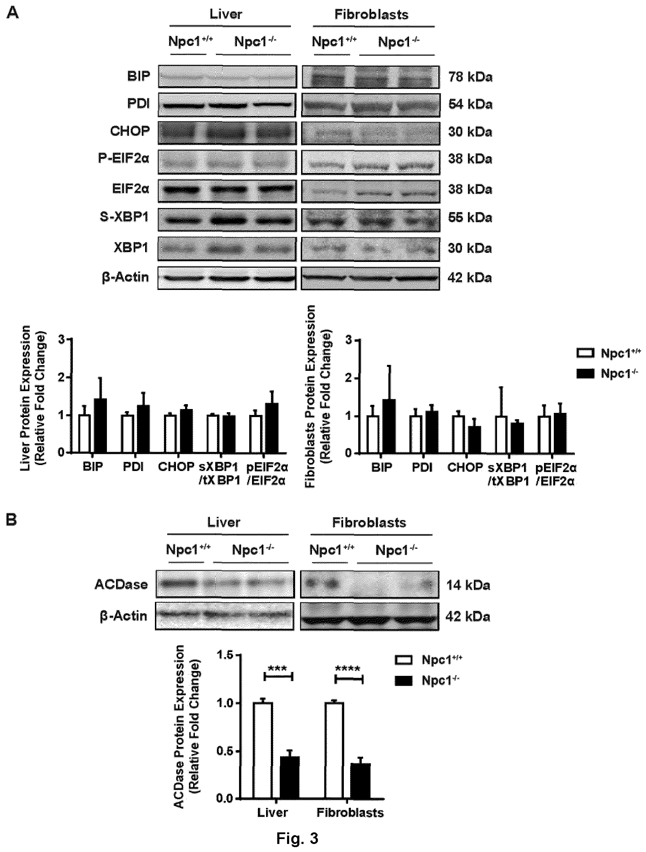

**Figure 3****. ACDase and ER stress markers protein expression in livers from Npc1⁻¹⁻ mice and fibroblasts from NPC patients.** A) ER stress markers and (B) ACDase protein expression analyzed by Western blot. Data are presented as means ± SEM for mice and ± SD for fibroblasts (n>3, Unpaired Student's t-test (two-tailed)).

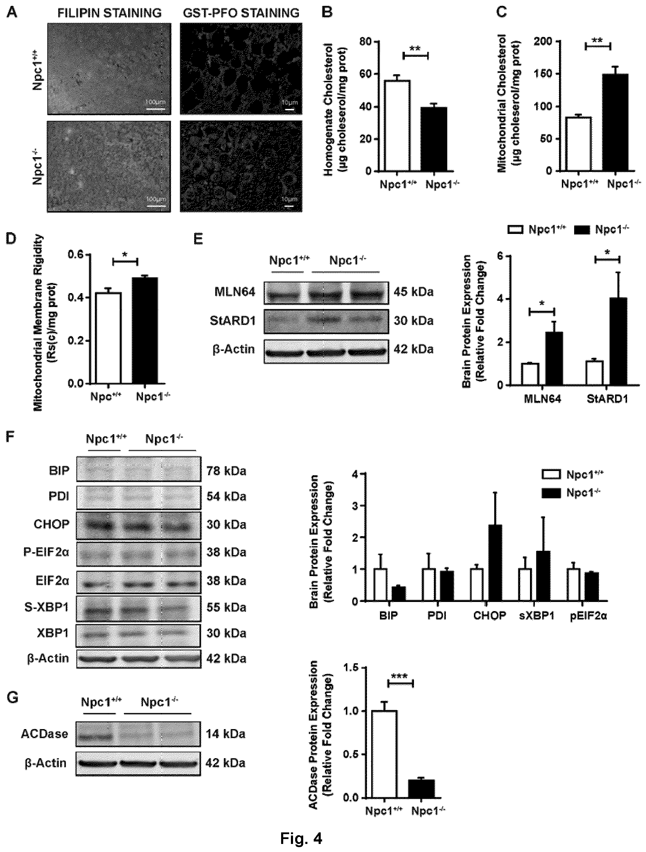

**Figure 4****. STARD1 upregulation and acid ceramidase downregulation in brain of Npc1^{-/-} mice.** Npc1^{+/+} and Npc1⁻¹⁻ mice were sacrificed at 6 weeks of age to isolate mitochondria from brain. (A) Filipin and GST-PFO staining. (B) Homogenate and (C) mitochondrial cholesterol levels by HPLC analyses. (D) Mitochondrial membrane order measured by DPH fluorescence anisotropy. (E) STARD1 and MLN64 cholesterol transporters, (F) ER stress markers and (G) ACDase protein expression analyzed by Western blot. Data are presented as means ± SEM (n>4, Unpaired Student's t-test (two-tailed)).

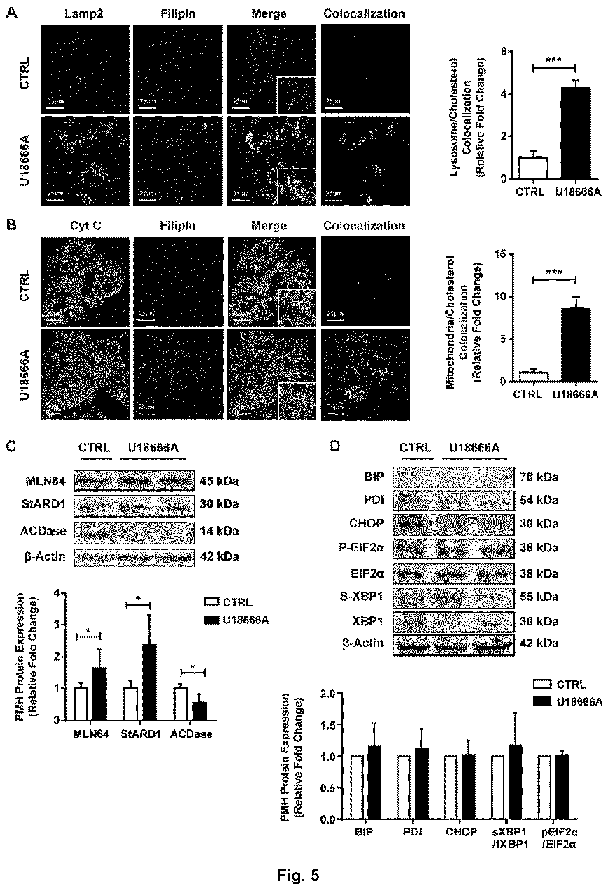

**Figure 5****. Effects of U18666A treatment on liver mitochondrial cholesterol trafficking.** Primary mouse hepatocytes from Npc1^{+/+} were treated with U18666A (2µg/µl, 16h) to determine (A) Lysosomes and Filipin co-staining, (B) Mitochondria and Filipin co-staining for confocal microscopy analyses. Staining markers colocalization analysis using Image J software. (C) STARD1, MLN64, ACDase and (D) ER stress markers protein expression analyzed by Western blot. Data are presented as means ± SD (n>5, Unpaired Student's t-test (two-tailed)).

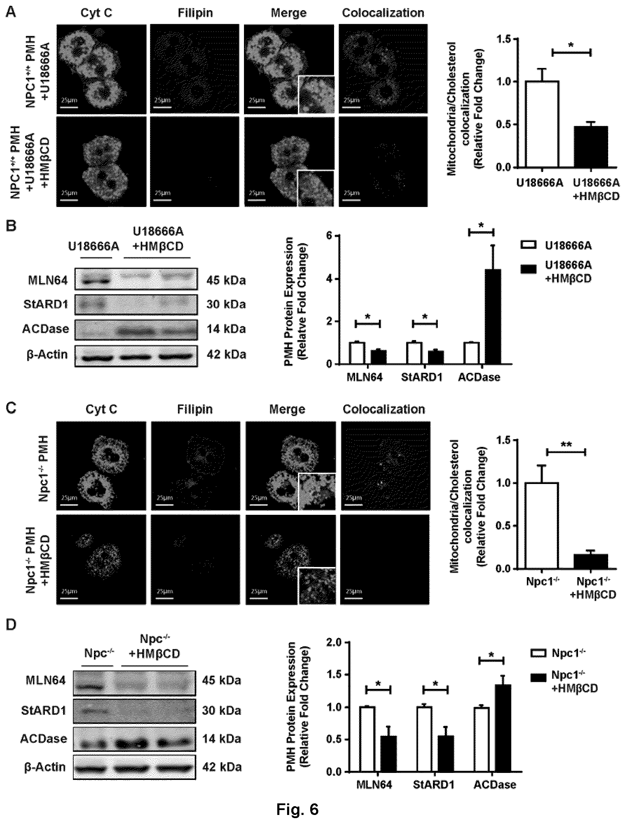

**Figure 6****. Effects of HMβCD on hepatocytes from Npc1+/+ mice treated with U18666A.** Primary mouse hepatocytes from Npc1^{+/+} treated with U18666A (2µg/µl, 16h) and primary mouse hepatocytes from Npc1^{-/-} were co-treated with HMβCD (2mM, 16h) to determine (A-C) Mitochondria and Filipin co-staining for confocal microscopy analyses. Staining markers colocalization analysis using ImageJ software. (B-D) STARD1, MLN64 and ACDase protein expression analyzed by Western blot. Data are presented as means ± SD (n>5, Unpaired Student's t-test (two-tailed)).

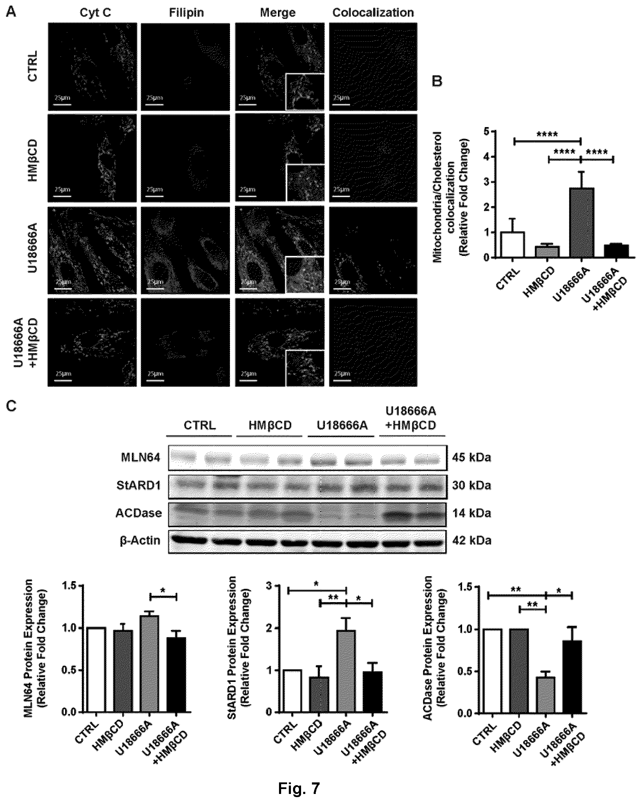

**Figure 7****. Effects of U18666A on mitochondrial cholesterol trafficking and HMβCD modulation in human fibroblasts.** Fibroblasts from control patients treated with U18666A (2µg/µl, 16h) were co-treated with HMβCD (2mM, 16h) to determine (A) Mitochondria and Filipin co-staining for confocal microscopy analyses. (B) Staining markers colocalization analysis using ImageJ software. (C) STARD1, MLN64 and ACDase protein expression analyzed by Western blot. Data are presented as means ± SD (n>5, Unpaired Student's t-test (two-tailed)).

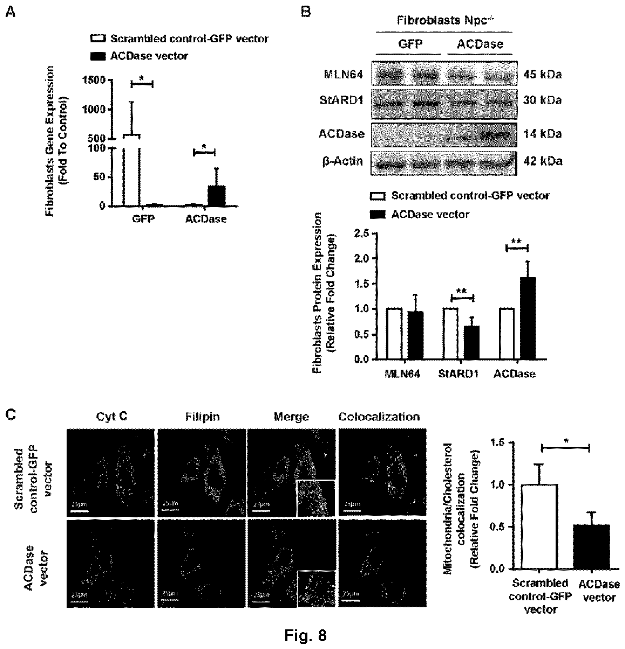

**Figure 8****. ACDase overexpression in fibroblasts from NPC patients.** Fibroblasts from NPC patients were transfected with an overexpression ACDase vector or the scrambled control-GFP vector to analyze (A) GFP and ACDase mRNA expression and (B) STARD1, MLN64 and ACDase protein expression by Western blot. (C) Mitochondria and Filipin co-staining for confocal microscopy analyses. Staining markers colocalization analysis using ImageJ software. Data are presented as means ± SD (n>5, Unpaired Student's t-test (two-tailed)). (D) LRH expression in fibroblasts from patients with NPC disease. Data are presented as means ± SD (Unpaired Student's t-test (two-tailed)).

### Examples

### Materials and Methods

### Npc1^{-/}⁻ model

Npc1^{-/-} mice (NPC1NIH, BALB/cJ strain) were obtained from The Jackson Laboratories. At the time of weaning (21 days), mice were genetically identified by PCR using DNA prepared from tail-tips and following the genotyping protocols provided by the supplier, as described previously in Torres et al., Redox Biol. 2017 Apr;11:60-72. All procedures involving animals and their care were approved by the Ethics Committee of the University of Barcelona and were conducted in accordance with institutional guidelines in compliance with national and international laws and policies.

### Fibroblasts from patients with NPC disease

Fibroblasts from patients with NPC disease used have been previously described (Torres et al., Redox Biol. 2017 Apr;11:60-72). Briefly, cultured human skin fibroblasts from control individuals (HSF; GM5659D) and patients with NPC disease were a generous gift from Thierry Levade, Laboratoire de Biochimie Metabolique, Institut Federatif de Biologie (CHU Toulouse, France) or obtained from Coriell Institute for Medical Research (GM03123, NJ, USA) and were grown at 37°C in 5% CO2. DMEM (Gibco) culture medium was supplemented with 10% fetal bovine serum (FBS, Gibco, 10-270-106) and 10.000U/ml Penicillin-Streptomycin (Gibco, 15140-122).

### Primary mouse hepatocytes (PMH) isolation

PMH were isolated by collagenase perfusion at a flow rate of 7-9ml/min and cultured at a density of 2×105 cells in 12-well plates coated with rat tail collagen. Hepatocytes were cultured in DMEM/F12 culture media (Gibco, 21331-020) supplemented with 10% FBS for the first 3 hours post isolation, together with 10.000U/ml Penicillin-Streptomycin (Gibco, 15140-122), 200mM L-Glutamine (Gibco, 25030-024), 7.5mM D-Glucose (Sigma, G6152) and 150mM Hepes pH 7.4 (Sigma,). For subsequent treatments, FBS was replaced for 1mM methionine (Sigma, M5308).

### In vitro treatments

PMH from Npc1^{+/+} and human Npc^{+/+} control fibroblasts were treated with U18666A (2µg/ml) or HMβCD (2mM) for 16 hours. In some other cases, PMH from Npc1^{-/-} and Npc^{-/-} fibroblast from NPC patients were treated with HMβCD as above for control PMH.

### ACDase overexpression

Fibroblasts from NPC patients were transfected with cDNA to overexpress ACDase (ASAH1) (SEQ ID NO: 5; MGC Human ASAH1 Sequence-Verified cDNA Accession: BC016481 Clone ID: 3923451) commercially purchased from GE Dharmacon. Transfection was performed using Lipofectamine2000 (Invitrogen). Briefly, 250000 fibroblasts were incubated with the transfection mixtures containing 250ng of the sobrexpression ACDase vector or the scrambled control-GFP vector. Cells were assayed 48 h after transfection for mRNA and protein levels.
SEQ ID NO: 5
>ENA|BC016481|BC016481.1 Homo sapiens N-acylsphingosine amidohydrolase (acid ceramidase) 1, mRNA (cDNA clone MGC:9327 IMAGE:3923451), complete cds.

CTGGAGTCCGGGGAGTGGCGTTGGCTGCTAGAGCGATGCCGGGCCGGAGTTGCGTCGCCT
TAGTCCTCCTGGCTGCCGCCGTCAGCTGTGCCGTCGCGCAGCACGCGCCGCCGTGGACAG

AGGACTGCAGAAAATCAACCTATCCTCCTTCAGGACCAACGTACAGAGGTGCAGTTCCAT
GGTACACCATAAATCTTGACTTACCACCCTACAAAAGATGGCATGAATTGATGCTTGACA
AGGCACCAATGCTAAAGGTTATAGTGAATTCTCTGAAGAATATGATAAATACATTCGTGC
CAAGTGGAAAAGTTATGCAGGTGGTGGATGAAAAATTGCCTGGCCTACTTGGCAACTTTC
CTGGCCCTTTTGAAGAGGAAATGAAGGGTATTGCCGCTGTTACTGATATACCTTTAGGAG
AGATTATTTCATTCAATATTTTTTATGAATTATTTACCATTTGTACTTCAATAGTAGCAG
AAGACAAAAAAGGTCATCTAATACATGGAGAAACATGGATTTTGGAGTATTTCTTGGGT
GGAACATAAATAATGATACCTGGGTCATAACTGAGCAACTAAAACCTTTAACAGTGAATT
TGGATTTCCAAAGAAACAACAAAACTGTCTTCAAGGCTTCAAGCTTTGCTGGCTATGTGG
GCATGTTAACAGGATTCAAACCAGGACTGTTCAGTCTTACACTGAATGAACGTTTCAGTA
TAAATGGTGGTTATCTGGGTATTCTAGAATGGATTCTGGGAAAGAAAGATGCCATGTGGA
TAGGGTTCCTCACTAGAACAGTTCTGGAAAATAGCACAAGTTATGAAGAAGCCAAGAATT
TATTGACCAAGACCAAGATATTGGCCCCAGCCTACTTTATCCTGGGAGGCAACCAGTCTG
GGGAAGGTTGTGTGATTACACGAGACAGAAAGGAATCATTGGATGTATATGAACTCGATG
CTAAGCAGGGTAGATGGTATGTGGTACAAACAAATTATGACCGTTGGAAACATCCCTTCT
TCCTTGATGATCGCAGAACGCCTGCAAAGATGTGTCTGAACCGCACCAGCCAAGAGAATA
TCTCATTTGAAACCATGTATGATGTCCTGTCAACAAAACCTGTCCTCAACAAGCTGACCG
TATACACAACCTTGATAGATGTTACCAAAGGTCAATTCGAAACTTACCTGCGGGACTGCC
CTGACCCTTGTATAGGTTGGTGAGCACACGTCTGGCCTACAGAATGCGGCCTCTGAGACA
TGAAGACACCATCTCCATGTGACCGAACACTGCAGCTGTCTGACCTTCCAAAGACTAAGA
CTCGCGGCAGGTTCTCTTTGAGTCAATAGCTTGTCTTCGTCCATCTGTTGACAAATGACA
GATCTTTTTTTTTCCCCCTATCAGTTGATTTTTCTTATTTACAGATAACTTCTTTAGGGG
AAGTAAAACAGTCATCTAGAATTCACTGAGTTTTGTTTCACTTTGACATTTGGGGATCTG
GTGGGCAGTCGAACCATGGTGAACTCCACCTCCGTGGAATAAATGGAGATTCAGCGTGGG
TGTTGAATCCAGCACGTCTGTGTGAGTAACGGGACAGTAAACACTCCACATTCTTCAGTT
TTTCACTTCTACCTACATATTTGTATGTTTTTCTGTATAACAGCCTTTTCCTTCTGGTTC
TAACTGCTGTTAAAATTAATATATCATTATCTTTGCTGTTATTGACAGCGATATAATTTT
ATTACATATGATTAGAGGGATGAGACAGACATTCACCTGTATATTTCTTTTAATGGGCAC
AAAATGGGCCCTTGCCTCTAAATAGCACTTTTTGGGGTTCAAGAAGTAATCAGTATGCAA
AGCAATCTTTTATACAATAATTGAAGTGTTCCCTTTTTCATAATTACTCTACTTCCCAGT
AACCCTAAGGAAGTTGCTAACTTAAAAAACTGCATCCCACGTTCTGTTAATTTAGTAAAT
AAACAAGTCAAAGACTTGTGGAAAATAGGAAGTGAACCCATATTTAAATTCTCATAAGT
AGCATTCATGTAATAAACAGGTTTTTAGTTTGTTCTTCAGATTGATAGGGAGTTTTAAAG
AAATTTTAGTAGTTACTAAAATTATGTTACTGTATTTTTCAGAAATCCAACTGCTTATGA
AAAGTACTAATAGAACTTGTTAACCTTTCTAACCTTCACGATTAACTGTGAAATGTACGT

CATTTGTGCAAGACCGTTTGTCCACTTCATTTTGTATAATCACAGTTGTGTTCCTGACAC
TCAATAAACAGTCATTGGAAAAAAAAAAAAAAAAAAAAAAAAAAAAAA

### Real Time PCR

Total RNA was isolated from fibroblasts with Trizol reagent following original protocol from Trizol's Reagent (manufacter's protocol). Quantitative Reverse Transcription Polymerase Chain Reaction (qRT-PCR) was performed using the iScript One-Step RT-PCR Kit with SYBR Green (Bio-Rad, Hercules, CA) following the manufacturer's instructions. Each reaction was run in duplicate to determine the threshold (CT) for each mRNA, and the amount of each cDNA relative to the β-Actin endogenous control was determined using the 2-ΔΔCt method. The primer sequences synthesized from Invitrogen used are displayed in Table 1:

**Table 1: Primer sequences used**

| **Gene** | **Primer** | **SEQ ID** | **Sequence** |
|---|---|---|---|
| *GFP* | **Forward primer (5'-3')** | SEQ ID NO: 6 | CAGGAGCGCACCATCTTCTT |
| | **Reverse primer (5'-3')** | SEQ ID NO: 7 | CTTGTGCCCCAGGATGTTG |
| *ASAH1 (ACDase)* | **Forward primer (5'-3')** | SEQ ID NO: 8 | AGTTGCGTCGCCTTAGTCCT |
| | **Reverse primer (5'-3')** | SEQ ID NO: 9 | TGCACCTCTGTACGTTGGTC |
| *ACTB* | **Forward primer (5'-3')** | SEQ ID NO: 10 | TTGCCGACAGGATGCAGAA |
| | **Reverse primer (5'-3')** | SEQ ID NO: 11 | GCCGATCCACACGGAGTACT |

### Recombinant GST-PFO probe

To assess an effective localization of cholesterol in membranes of liver, brain and liver tissues, we used a recombinant Perfringolysin O (PFO) fusioned with Glutathione-S-Transferase (GST-PFO). In tissues, PFO is able to track cholesterol of outer and inner membranes when these have more than 30% mol of cholesterol and the fusion with GST allows the detection by immunofluorescence techniques. The production of recombinant GST-PFO probe has been described in (Kwiatkowska, K et al. Orphanet J Rare Dis 2014; 9 (1) 64). The sequence to design the plasmid was from NCBI database: DNA M36704, the gene of PFO in the *Clostridium perfringens* genome. Additionally, the signal peptide was eliminated from the PFO gen to enable the intracellular stance of the protein. The resulted sequence was synthesized into the plasmid pGEX 4T-1 (GenScript) between the *Bam*HI and *Sma*I restriction sites. The plasmid was engineered to make a fusion protein with the GST tag. After bacterial production, and protein extraction and purification, the GST-PFO probe was dialyzed, concentrated, quantified and stored at -80°C.

### Filipin and GST-PFO staining

Cryopreserved liver, brain and cerebellum samples from were cut on a cryostat at 14 micrometers thin sections and placed on glass slides. Sections were brought to room temperature for 30 min, fixed in 4% paraformaldehyde for 20 minutes and washed with PBS. For Filipin staining tissues were incubated with 25µg/mL Filipin (Sigma) over night at 4°C protected from the light. After washing, slices were mounted with prolong antifade mounta nt (Dako). For GST-PFO staining, the sections were permeabilized with 0.2% Triton X-100 in blocking buffer (5% goat serum + 1% BSA in PBS) for 2 h in a dark-humid chamber. Then, slices were incubated 3 h with the probe GST-PFO (20µg/ml) in 1% Goat Serum containing 0.05% Triton X-100 in PBS. After washing ×3 with PBS, samples were incubated O.N. at 4°C with primary antibody Glutathione-STransferase (GST) (Santa Cruz). Secondary antibodies were diluted 1:200 in 1% Goat Serum containing 0.05% Triton X-100 in PBS and incubated for 90 min at RT with the mix of anti-mouse Alexa fluor-532 (for GST). After washing, slices were incubated 5 min in sudan black 0.1% in 70% EtOH to minimize autofluorescence and mounted with prolong antifade mountant (Dako). Images for all samples were taken with a Leica TCS SP5 laser scanning confocal system with a 63x oil immersion objective APO CS numerical aperture 1.4 equipped with a DMI6000 inverted microscope.

### Mitochondria isolation

Mitochondrial fraction was isolated from liver and brain by Percoll density grandient centrifugation as described previously (Torres et al., Redox Biol. 2017 Apr; 11:60-72). Mitochondrial purity and cross contamination with extramitochondrial compartments was checked as described previously (Fernandez, A et al. J. Hepatol. 2013;59(4):805-813). Alternatively, PMH and fibroblasts were fractionated into cytosol and mitochondria by digitonin permeabilization as described previously (Fernandez, A et al. J. Hepatol. 2013;59(4):805-813).

### Cholesterol measurements

Total cholesterol determination in homogenates (1mg/ml) or mitochondrial extracts (2.3 mg/ml) was performed upon saponification with alcoholic KOH followed by water:hexane 1:2 extraction. Hexane phase was evaporated in a speed vacuum and used for cholesterol measurement. Free cholesterol was determined from the same unsaponified samples and analyzed by HPLC using Bondapak C18 10µm reversed-phase column (30cm x 4mm inner diameter; Waters, Cromatografia, S.A., Barcelona, Spain), and 2-propanol/acetonitrile/water (60:30:10) as mobile phase at a flow rate of 1ml/min as described. The amount of cholesterol was calculated from standard curves and the identity of the peaks was confirmed by spiking the sample with known standards.

### Measurement of fluorescence anisotropy

Fluidity of mitochondrial membranes was evaluated by fluorescence anisotropy of mitochondria-bound dye DPH. DPH (20 mM in tetrahydrofuran) was first diluted 100 times with 10 mM Tris-HCI, 150 mM KCI, 1 mM EDTA, pH 7.4. Subsequently, DPH was injected into stirred mitochondrial suspensions (0.5 mg/ml) and the mixture was incubated for 30 min at 37 °C. Fluorescence polarization was measured in a Hitachi spectrofluorometer at wavelengths of 366 nm for excitation and 425 nm for emission. The results are expressed as anisotropy units (r), where r = (I0/I90)/(I0+2I90). I0 and I90 represent the intensities of light when polarizers were in parallel or perpendicular orientation, respectively. Light scattering and intrinsic fluorescence were routinely corrected by subtracting the signal obtained from unlabeled samples and the fluorescence of the buffer plus label alone.

### ROS generation and cell viability

For ROS production and cell viability assays, fibroblasts were first treated with hydrogen peroxide (H2O2) 1mM for 24 hours. For ROS production analysis, cells were then incubated in DCF (10µM) solution for 30 minutes at 37°C in dark, washed with PBS and evaluated by fluorescence spectroscopy using the Tecan Infinite^{®} 200 PRO multi-mode microplate reading (485nm for excitation and 528nm for emission). The fluorescence was normalized by protein content, determined by BCA protein assay. The induction of reactive species was calculated with respect to control samples. For cell viability analysis, cells were then incubated with Thiazolyl Blue Tetrazolium Bromide (MTT) (1,2mM) solution for 30 minutes at 37°C in dark. Medium was subsequently aspirated and cells were resuspended in 1-propanol and evaluated by fluorescence spectroscopy (570nm for formazan and 630nm as background). Viability was finally calculated as absorbance (570-630) x 100/ absorbance control (570-630).

### Western blot analysis

100mg of liver were homogenized with a pestle homogenizer in 1ml of homobuffer (70mM saccharose, 220mM mannitol, 2mM Tris-HCI pH 7.4, 0,1mM EDTA, 0.1% fatty acid free BSA) supplemented with protease and phosphatase inhibitors (Roche). Liver homogenates were diluted 4 times with RIPA lysis buffer (Sigma) with anti-proteases and anti-phosphatases. Cells were washed with PBS and lysed with ice-cold RIPA lysis buffer supplemented with protease and phosphatase inhibitors. Samples were incubated 15min at 4°C, vortexed and spinned down for 5min at 10000rpm. All supernatants were collected and quantified for protein concentration using Quick-Bradford reagent (Bio-Rad) and 20-50µg of protein were subjected to 4-12% SDS-polyacrylamide gel electrophoresis (SDS-PAGE) (Bio-Rad, XT-Criterion). Proteins were electrotransferred at 4°C onto Hybond ECL nitrocellulose membranes (Amersham) and 5% BSA solution in TBS-Tween was used to block the membranes for 1h at room temperature. Membranes were incubated overnight with the following primary antibodies: anti-PDI (Cell Signaling, 24465), anti-CHOP (Cell Signaling, 2895S), anti-Phospho eiF2A (Cell Signaling, 9721S), anti-eiF2A (Cell Signaling, 9722) and anti-STARD1 (Abcam, ab58013), anti-BIP (Stressgen, SPA826), anti-XBP1 (Santa Cruz, CS-8015) and anti-Mln64 (Santa Cruz SC-292868), ACDAse (Sigma, SAB3500293), LRH-1 (Santa Cruz, Cs-393369) and anti-β-Actin HRP-conjugated (Sigma, A3854). Membranes were thoroughly washed with TBS-Tween and incubated for 45min with their respective HRP-conjugated secondary antibodies. Pierce ECL Western Blotting Substrate (Thermo Scientific) was used to develop the membranes. Images were digitally captured by LAS4000 (GE Healthcare) and optical density was analyzed with ImageJ software.

### Immunofluorescence and laser confocal imaging

PMH were fixed for 15min with 3.7% paraformaldehyde and permeabilized for 5min with 0.2% saponin dissolved in 0.5% BSA-fatty acid free (FAF) in PBS commercial buffer. Cells were blocked with 1% BSA FAF for 15minutes. Cytochrome c (Cytc) (BD Pharmigen, #556432) and Lamp2 (Abcam, #ab13524) primary antibodies were incubated overnight in BSA 1% followed by a secondary antibody for 1 hour at room temperature in BSA 0.1%. Filipin (50 mg/ml, Sigma) was added during the secondary antibody incubation and the following steps were performed in the dark. Stained samples were embedded in fluoromont (Sigma) and digital images were taken in a Leica SP2 laser scanning confocal microscope equipped with UV excitation, an argon laser, a 633/1.32 OIL PH3 CS objective and a confocal pinhole set at 1 Airy unit. All the confocal images shown were single optical sections. Scale bar represents 25 µm. Percentage of cholesterol mass containing mitochondria was analyzed with the Colocalization nBits nimages plugin (Confocal Microscopy Unit, Facultad de Medicina, Universidad de Barcelona) in the ImageJ Software in 5 consecutive images of each experimental condition.

### Extracellular flux analyses

In vivo real-time mitochondrial respiration (OCR) was monitored with the Seahorse XFe24 Flux Analyser (Seahorse Bioscience) according to the manufacturer's instructions. Fibroblasts were seeded at 20000 fibroblasts/well density in 24-well plates and cultured overnight in DMEM culture media. For assessment of the real-time OCR, cells were incubated with unbuffered assay media (XF Media Base with 10mM glucose, 1mM L-glutamine and 1mM sodium pyruvate) followed by a sequential injection of 2µM oligomycin, 1µM carbonyl cyanide-4-(trifluoromethoxy) phenylhydrazone and 1µM antimycin A plus Rotenone. OCR measurements were normalized to µg of total protein following BCA protein assay.

### Statistical Analyses

Statistical analyses were performed using GraphPad Prism 6 (Graphpad Software Inc). Unpaired Student's t-test (two tailed) was performed between two groups and one or two-way ANOVA followed by Tukey's Multiple Comparison test were used for statistical comparisons between three or more groups, with p<0.05 considered as significant (*= p<0.05, **= p<0.01, ***= p<0.001, ****= p<0.0001). The corresponding number of experiments is indicated in the figure legends. Data in graphs are shown as mean±s.e.m.

### Example 1: Hepatic mitochondrial cholesterol accumulation and STARD1 upregulation in Npc1^{-/-}mice

Accumulation of cholesterol in lysosomes caused by mutations in NPC1 is a hallmark of NPC disease. Besides this event, accumulation of cholesterol in mitochondria of affected organs from Npc1^{-/-} mice has also been initially reported and not well characterized (Yu W et al. J. Biol. Chem. 2005;280(12):11731-11739). Hence, before exploring potential mechanisms, we further analyzed hepatic mitochondrial cholesterol homeostasis in Npc1⁻¹⁻ mice. First, staining of liver sections with GST-PFO, which detects free cholesterol levels in membranes, increased in liver from Npc1^{-/-} mice compared to Npc1^{+/+} mice, with similar findings seen with filipin staining (Figure 1A). These results were further confirmed by HPLC analyses of liver homogenates and isolated mitochondrial fraction from Npc1^{-/-} mice (Figure 1B-C). Consistent with the increase in mitochondrial cholesterol loading, fluorescence anisotropy analysis of liver mitochondria labeled with the fluorescent probe DPH revealed an increase in mitochondrial membrane order from Npc1-/- mice compared to Npc1^{+/+} mice (Figure 1D). In addition, confocal microscopy analyses of primary mouse hepatocytes (PMH) from 6-week-old Npc1^{-/-} mice indicated increased filipin staining and colocalization with cytochrome c-labeled mitochondria (Figure 1E, F), indicating the increase in mitochondrial cholesterol levels. As a potential mechanism accounting for the increase in mitochondrial cholesterol content, we examined the expression of STARD1, which plays a crucial role in the intramitochondrial cholesterol trafficking from the outer to inner mitochondrial membrane for metabolism. Remarkably, STARD1 expression increased 3-fold at the protein and mRNA level in liver of Npc1^{-/-} mice (Figure 1G). Moreover, the levels of MLN64, an endosomal member of the STAR family involved in egress of cholesterol from endosomes to STARD1 in the mitochondrial outer membrane, increased in liver of Npc1^{-/-} mice compared to Npc1^{+/+} mice (Figure 1G). These findings establish the increase in hepatic mitochondrial cholesterol levels that correlate with expression of STARD1 and MLN64.

### Example 2: Fibroblasts from patients with NPC disease exhibit increased mitochondrial cholesterol levels and STARD1 expression

To validate the relevance of the preceding findings to human disease, we examined the homeostasis of mitochondrial cholesterol in fibroblasts from patients with NPC disease. In line with findings in liver, fibroblasts from patients with NPC disease (Npc^{-/-}) exhibited increased free cholesterol levels determined by HPLC analysis in homogenate and isolated mitochondrial fractions compared to fibroblasts from control subjects (Npc^{+/+}) (Figure 2A-B). Furthermore, confocal microscopy analyses of Npc^{-/-} fibroblasts revealed mitochondrial free cholesterol accumulation with respect to Npc^{+/+} fibroblasts, as indicated by the colocalization of filipin and cytochrome c staining (Figure 2C-D). Moreover, STARD1 and MLN64 expression also increased in Npc^{-/-} fibroblasts from patients with NPC disease compared to control Npc^{+/+} fibroblasts (Figure 2E). Thus, these findings confirm the outcome observed in liver from Npc1^{-/-} mice and reveal the mitochondrial cholesterol accumulation in human NPC disease.

### Example 3: Livers from Npc1^{-/-} mice and fibroblasts from NPC patients exhibit decreased ACDase expression without evidence of ER stress.

Given the relevance of STARD1 in the trafficking of cholesterol to mitochondrial inner membrane, we next examined potential mechanisms involved in the upregulation of STARD1 in NPC disease. ER stress has been shown to stimulate the transcriptional upregulation of STARD1 independently of SREBP activation, and hence we analyzed the expression of ER stress markers in liver from Npc1^{-/-} mice and Npc^{-/-} fibroblasts from patients with NPC disease. While tunicamycin induced markers of ER stress (BIP, PDI and CHOP) in control Npc^{+/+} fibroblasts, the levels of ER stress markers BIP, PDI, CHOP, p-EIF2α and s-XBP1 in liver fractions from Npc1^{-/-} mice or Npc^{-/-} fibroblasts from patients with NPC disease were similar to those seen in Npc1^{+/+} mice or control Npc^{+/+} fibroblasts (Figure 3A). Given these findings, we searched for alternative mechanisms involved in STARD1 regulation. As STARD1 is also regulated by steroidogenic factor 1 (SF-1), a member of the nuclear receptor family, and ACDase has been shown to repress SF-1 and subsequently STARD1 upregulation, we next examined ACDase expression in livers from Npc1^{-/-} mice and Npc^{-/-} fibroblasts from patients with NPC disease. Interestingly, the expression of ACDase decreased in liver or PMH from Npc1^{-/-} mice and Npc^{-/-} fibroblasts from NPC patients (Figure 3B) and was confirmed by confocal microscopy analyses. Moreover, as liver receptor homolog 1 (LRH-1), another member of the nuclear receptor family highly related to SF-1, regulates STARD1, we examined its expression in NPC disease. As seen, LRH-1 levels increased in liver from Npc1^{-/-} mice and in Npc^{-/-} fibroblasts from NPC patients. Thus, these data revealed for the first time an inverse correlation between ACDase and STARD1 expression in NPC disease.

### Example 4: Mitochondrial cholesterol status and expression of STARD1 and ACDase in brain and cerebellum from Npc1^{-/-} mice.

We next examined the cholesterol homeostasis in brain homogenate from *Npc1⁻¹⁻* mice. In contrast to liver, brain homogenate from *Npc1*^{-/-} mice exhibited lower free cholesterol levels compared to *Npc1*^{*+*/*+*} mice, as revealed by filipin and GST-PFO staining or HPLC analyses (Figure 4A, B), with similar findings observed in cerebellum from *Npc1*^{-/-} mice. Since myelin is the major source of cholesterol in the brain, these findings are consistent with previous reports showing that in NPC disease cholesterol accumulates in most tissues but not the brain due to the extensive demyelination that occurs in this disease. Quite interestingly, however, despite lack of increase of cholesterol levels in brain homogenate, isolated brain mitochondria exhibited increased mitochondrial cholesterol accumulation that resulted in increased mitochondrial membrane order (Figure 4C, D), indicating the activation of a specific mechanism leading to increased mitochondrial cholesterol trafficking. Indeed, expression of STARD1 increased 4-fold in brain and cerebellum from *Npc1*^{-/-} mice (Figure 4E). MLN64 expression increased as well although to a lower extent than STARD1 (Figure 4E). In agreement with findings in liver, brain or cerebellum from *Npc1*^{-/-} mice failed to exhibit signs of ER stress as revealed by the expression of BIP, PDI, CHOP, sXBP-1 and p-EIF2 (Figure 4F). However, decreased levels of ACDase were observed in brain or cerebellum from *Npc1*^{*-*/*-*} mice (Fig 4G), in line with findings in liver. Overall, these findings confirm the accumulation of mitochondrial cholesterol in brain and cerebellum from *Npc1*^{*-*/*-*} mice and further establish an inverse relationship between STARD1 and ACDase in these affected organs.

### Example 5: The amphiphilic cationic sterol U18666A reproduces the inverse correlation between STARD and ACDase in Npc1^{+/+} PMH.

We next further characterized the relationship between STARD1 and ACDase. Since ACDase is a lysosomal enzyme, we first addressed whether the decrease in ACDase expression in NPC cells could be a consequence of the accumulation of cholesterol in lysosomes. Hence, we used the amphiphilic aminosteroid U186661, which antagonizes NPC1 and increases intracellular cholesterol accumulation. Confocal imaging analysis of PMH from *Npc1*^{*+*/*+*} mice stained with filipin and Lamp2 revealed that U18666A significantly increased lysosomal cholesterol accumulation, reproducing the primary feature of NPC disease (Figure 5A). In addition, U18666A also stimulated the accumulation of cholesterol in mitochondria, as seen by the colocalization of filipin and CytC staining of *Npc1*^{*+*/*+*} PMH (Figure 5B), in line with the findings seen in *Npc1*^{-/-} PMH. Moreover, similar accumulation of mitochondrial cholesterol was observed in *Npc*^{+/+} fibroblasts upon U18666A treatment (Figure 6A-B). Interestingly, U18666A treatment significantly increased STARD1 in both PMH from *Npc1*^{*+*/*+*} mice (Figure 5C) and *Npc*^{+/+} fibroblasts (Figure 6C). Similar findings were observed in the expression of MNL64 although to a lower extent than that of STARD1 (Figure 5C; Figure 6C). Moreover, in line with preceding findings in liver or brain tissues, U18666A treatment decreased the expression of ACDase in PMH from *Npc1*^{*+*/*+*} mice (Figure 5C) or control Npc^{+/+} fibroblasts (Figure 6C). In addition, incubation of *Npc1*^{*+*/*+*} PMH with U18666A failed to induce ER stress markers (Figure 5D), in line with findings in *Npc1*^{*-*/*-*} PMH. Thus, these findings provide evidence for a link between lysosomal cholesterol accumulation with ACDase repression and enhanced STARD1 expression.

### Example 6: 2-hydroxypropyl-β-cyclodextrin prevents U18666A-mediated ACDase downregulation and STARD1 upregulation.

To further establish the role of cholesterol in the relationship between STARD1 and ACDase, we next examined the impact of 2-hydroxypropyl-β-cyclodextrin (HMβCD) in the expression of ACDase and STARD1 in response to U18666A. Treatment of Npc^{+/+} fibroblasts from control patients or PMH from *Npc1*^{*+*/*+*} mice with HMβCD prevented U18666A-induced cholesterol accumulation and trafficking to mitochondria in both cell types (Figure 6A-B and Figure 7A). Moreover, this outcome paralleled the ability of HMβCD to reverse the increase of STARD1 and the repression of ACDase induced by U18666A in control Npc^{+/+} fibroblasts and PMH from *Npc1*^{*+*/*+*} mice (Figure 6C and Figure 7B). In addition, PMH from *Npc1*^{*-*/*-*} mice and Npc^{-/-} fibroblasts from NPC patients exhibited the same changes induced by HMβCD regarding the reversed relationship between the expression levels of STARD1 and ACDase and the impact in mitochondrial cholesterol increase (Figure 7C-D). In addition to these changes, HMβCDprevented the increased expression of MLN64 induced by U18666A in PMH from *Npc1*^{*+*/*+*} mice or control *Npc*^{*+*/*+*} fibroblasts (Figure 6C and Figure 7B). These findings further establish a cholesterol-dependent relationship between STARD1 and ACDase.

### Example 7: ACDase overexpression in fibroblasts from NPC patients prevents STARD1 upregulation and mitochondrial cholesterol accumulation without alterations in MLN64 expression.

To examine the cause-and-effect relationship between the decreased expression of ACDase and the induction of STARD1, we analyzed whether ACDase overexpression has a specific impact in the regulation of STARD1. For this purpose, Npc^{-/-} fibroblasts from NPC patients were transfected with cDNA encoding ACDase (SEQ ID NO: 5; MGC Human ASAH1 Sequence-Verified cDNA Accession: BC016481). Compared to transfection with scrambled control GFP vector, ACDase transfection resulted in a 30-fold increase in ACDase expression (Figure 8A), which translated in significantly higher ACDase protein levels (Figure 8B). While this event barely affected MLN64 expression, ACDase overexpression markedly downregulated the expression of STARD1 in *Npc*^{*-*/*-*} fibroblasts (Figure 8B). Interestingly, given the role of LRH-1 in the transcriptional activation of STARD1, ACDase transfection repressed the expression of LRH-1 in *Npc*^{-/-} fibroblasts from patients with NPC disease. To determine the impact of ACDase overexpression on the trafficking and accumulation of cholesterol in mitochondria, we performed confocal microscopy analyses of *Npc*^{-/-} fibroblasts transfected with ACDase. *Npc*^{*-*/*-*} fibroblasts from patients with NPC disease exhibited lower mitochondrial free cholesterol levels as indicated by the colocalization of filipin with Cyt C staining, compared to scrambled control-GFP transfected fibrloblasts (Figure 8C). These findings indicate that ACDase expression represses STARD1 upregulation and decreases mitochondrial cholesterol accumulation in human NPC disease.

### Example 8: ACDase overexpression in fibroblasts from NPC patients increases mGSH, improves mitochondrial function and protects against oxidative stress and cell death.

We next examined the functional impact of ACDase overexpression in fibroblasts from NPC patients. Consistent with the ability of ACDase to decrease STARD1 expression and mitochondrial cholesterol accumulation (Figure 8), *Npc*^{*-*/*-*} fibroblasts from patients with NPC transfected with ACDase exhibited increased mGSH levels compared to fibroblasts transfected with control GFP vector. Moreover, ACDase expression increased real-time oxygen consumption rates determined by an extracellular flux analyser compared to *Npc*^{*-*/*-*} fibroblasts from NPC patients transfected with control GFP control vector, leading to enhanced rates of basal respiration, ATP production and maximal respiration. Furthermore, ACDase transfection also resulted in decreased oxidative stress in response to H₂O₂ challenge as revealed by lower dichlorofluorescein (DCF) fluorescence compared to *Npc*^{-/-} fibroblasts from NPC patients transfected with control GFP vector. In line with these observations, *Npc*^{-/-} fibroblasts from NPC patients transfected with control GFP vector were sensitive to H₂O₂-induced oxidative cell death and this outcome was ameliorated by ACDase transfection, consistent with the replenishment of mGSH levels and attenuation of DCF fluorescence. Overall, these findings indicate that expression of ACDase has important functional consequences in fibroblasts from NPC patients.

## Claims

1. A steroidogenic acute regulatory protein 1 (STARD1) inhibitor for use in medicine.

2. A STARD1 inhibitor for use in the treatment of lysosomal storage disorder in a subject.

3. A STARD1 inhibitor for use according to claim 1 or 2, wherein the lysosomal storage disorder is Niemann-Pick type C (NPC) disease.

4. A STARD1 inhibitor for use according to any one of claims 1 to 3, wherein the STARD1 inhibitor is selected from the group consisting of a chemical compound, a STARD1 specific siRNA, a STARD1 specific antisense oligonucleotide, a STARD1 specific ribozyme and a STARD1 specific antibody, and a protein regulating the STARD1 expression.

5. A STARD1 inhibitor for use according to claim 4, wherein the protein regulating the STARD1 expression is an acid ceramidase (ACDase) enzyme or a functionally equivalent fragment thereof.

6. A STARD1 inhibitor for use according to claim 5, wherein the ACDase enzyme comprises an amino acid sequence having an identity of at least 70% with the sequence SEQ ID NO: 1.

7. A STARD1 inhibitor for use according to any one of claims 1 to 6, wherein the STARD1 inhibitor is comprised within a pharmaceutical composition.

8. An *in vitro* method for diagnosing lysosomal storage disorder in a subject, or for designing a customized therapy for a subject suffering from lysosomal storage disorder, comprising:
(i) quantifying the expression levels of the nucleotide sequence encoding the STARD1 in a biological sample isolates from the subject, and
(ii) comparing the expression levels obtained in step (i) with a reference level,
wherein an increased STARD1 expression levels with respect to the reference level is indicative that the subject suffers from lysosomal storage disorder, or that the therapy to be administered to the subject is based on a STARD1 inhibitor.

9. An *in vitro* method according to claim 8, wherein the lysosomal storage disorder is selected from a list consisting of: GM1 gangliosidosis, GM2 gangliosidosis, Alpha-mannosidosis, Hurler syndrome, Hunter syndrome, Sanfilippo syndrome Type A, Morquio syndrome Type A, Morquio syndrome Type B, Niemann-Pick type A (NPA), Niemann-Pick type B (NPC B) or Niemann-Pick type C (NPC) disease.

10. An *in vitro* method according to claim 8 or 9, wherein the quantification of the expression levels comprises measuring the level of cDNA, the level of mRNA, and/or the level of the protein encoded by said nucleotide sequence.

11. An *in vitro* method according to any one of claim 8 to 10, wherein the subject is a mammal, preferably, the mammal is a human.

12. An *in vitro* method according to any one of claims 8 to 11, wherein the STARD1 inhibitor is a STARD1 inhibitor is selected from the group consisting of a chemical compound, a STARD1 specific siRNA, a STARD1 specific antisense oligonucleotide, a STARD1 specific ribozyme and a STARD1 specific antibody, and a protein regulating the STARD1 expression.

13. An *in vitro* method according to any one of claims 8 to 12, wherein the protein regulating the STARD1 expression is an acid ceramidase (ACDase) enzyme or a functionally equivalent fragment thereof.

14. An *in vitro* method according to claim 13, wherein the ACDase enzyme comprises an amino acid sequence having an identity of at least 70% with the sequence SEQ ID NO: 1.
